# EUROPEAN PATENT APPLICATION

(11) **EP 3 064 493 A1**
(43) Date of publication of application: **07.09.2016**
(21) Application number: 15158053.7
(22) Date of filing: 06.03.2015
(51) Int. Cl.: C07D 401/06, A61K 31/4709, A61P 35/00

(54) **CRYSTALLINE FORMS OF (R)-(2-(4-CHLOROPHENYL)QUINOLIN-4-YL)((S)-(PIPERIDIN-2-YL)METHANOL**

(71) Applicant: Glionova AB, 111 53 Stockholm (SE)
(72) Inventor: Bohlin, Martin, 151 36 SÖDERTÄLJE (SE); Kindstedt Danielsson, Ann-Sofi, 151 36 SÖDERTÄLJE (SE); Larsson, Ulf, 151 36 SÖDERTÄLJE (SE); Noreland, David, 151 36 SÖDERTÄLJE (SE); Svensson, Per H, 151 36 SÖDERTÄLJE (SE); Ymén, Ingvar, 151 36 SÖDERTÄLJE (SE); Åslund, Bengt, 151 36 SÖDERTÄLJE (SE); Hammarström, Lars, 111 53 STOCKHOLM (SE)
(74) Representative: Awapatent AB

(57) **Abstract**

The present invention relates to novel crystalline forms of Vacquinol-1, in particular novel crystalline forms of (R)-(2-(4-chlorophenyl)quinolin-4-yl)((S)-piperidin-2-yl)methanol and to methods for their preparation. The invention is also directed to pharmaceutical compositions containing at least one such crystalline form, therapeutic or prophylactic use of such crystalline forms and compositions and methods of preparing such crystalline forms.

## Description

### Field of the invention

The present invention relates to novel crystalline forms of Vacquinol-1, in particular novel crystalline forms of (R)-(2-(4-chlorophenyl)quinolin-4-yl)((S)-piperidin-2-yl)methanol and to methods for their preparation. The invention is also directed to pharmaceutical compositions containing at least one such crystalline form, therapeutic or profylactic use of such crystalline forms and compositions and methods of preparing such crystalline forms.

### Background of the invention

The present invention relates to novel crystalline forms of (R)-(2-(4-chlorophenyl)quinolin-4-yl)((S)-piperidin-2-yl)methanol. This compound represents a stereoisomer of the compound Vacquinol-1. Anti-cancer activity of the racemic Vacquinol-1 (NSC13316) has been shown in glioma cells (Kitambi, SS et al, Cell, 2014, 157(2), 313-28) and therefore it was hypothesized therein that racemic Vacquinol-1 could be useful in treatment of non-systemic CNS-related types of cancer, such as glioblastoma. Racemic Vaquinol-1 has the following molecular structure:

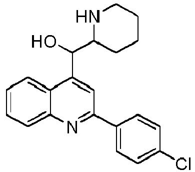

As understood by those skilled in the art, it is desirable to obtain crystalline forms with improved physical properties, compared to amorphous material and which are useful in in formulations that could be reliably used in manufacture and distribution. Such properties typically include improved stability towards heat, moisture and light, as well as optimized filterability and hygroscopy.

Polymorphs are stoichiometrically identical entities of a compound, which have different crystal structures. Since polymorphs have different crystal structures they also have different physical properties such as solubility, dissolution rate, melting point etc. The different crystal structures are clearly shown in the X-ray diffraction characteristics of the crystals.

Crystal modifications or crystal forms are the common term for crystalline ansolvates and solvates of a compound. Thus, an anhydrate and a monohydrate of a compound are different crystal modifications or different crystal forms of such a compound.

It is desireable to the pharmaceutical industry to obtain a crystalline material of a single crystal form, that provides a reliable dosage form, not the least during clinical studies, in order to provide reliable data. Further, during manufacture, optimized properties of a single crystal form is desireable in order to obtain reproducible physical properties such as solubility and dissolution properties (which in turn leads to reproducible bioavailability), improved chemical stability, reduced levels of impurities, enhanced thermodynamic stability and to minimal hygroscopic tendencies.

### Description of the invention

Racemic Vacquinol-1 contains a mixture of 4 stereoisomers, namely:
(R)-(2-(4-chlorophenyl)quinolin-4-yl)((S)-piperidin-2-yl)methanol (**S1**);
(S)-(2-(4-chlorophenyl)quinolin-4-yl)((R)-piperidin-2-yl)methanol (**S2**);
(R)-(2-(4-chlorophenyl)quinolin-4-yl)((R)-piperidin-2-yl)methanol (**S3**); and
(S)-(2-(4-chlorophenyl)quinolin-4-yl)((S)-piperidin-2-yl)methanol (**S4**). The structures of **S1**, **S2**, **S3** and **S4**, respectively, are shown in the Scheme 1 below.

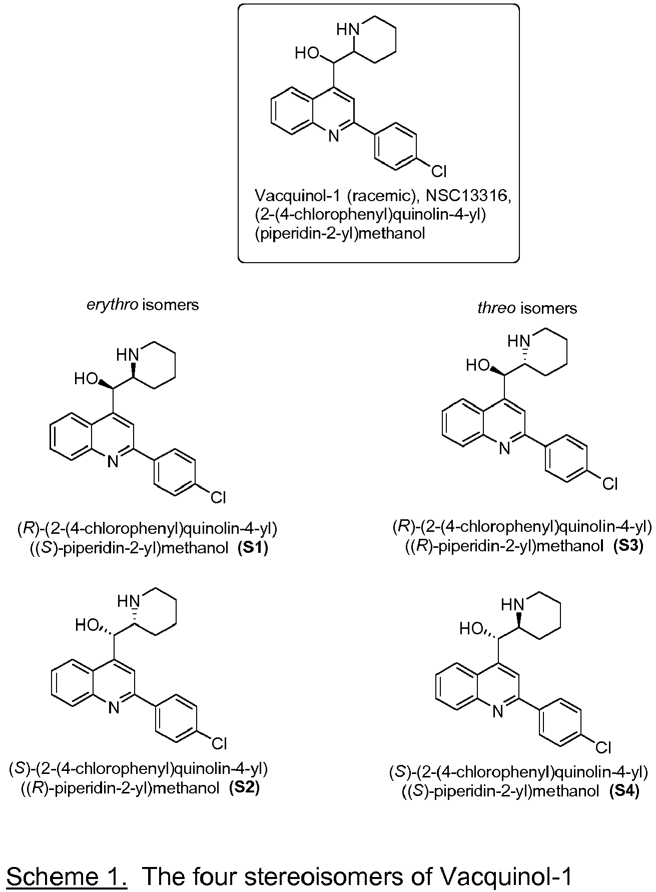

One aspect of the invention provides a crystal form of (R)-(2-(4-chlorophenyl)quinolin-4-yl)((S)-piperidin-2-yl)methanol represented by compound **S1**: 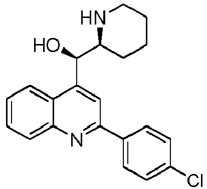 wherein said compound **S1** is in the form of an anhydrate (Form A1).

In one embodiment of this aspect, there is provided a crystal formof (R)-(2-(4-chlorophenyl)quinolin-4-yl)((S)-piperidin-2-yl)methanol (**S1**) in the form of an anhydrate (Form A1), wherein said crystal form has a powder X-ray diffraction pattern, as obtained with CuKα1-radiation, with characteristic peaks at diffraction angles (°2theta) of 10.4 ± 0.2 and 19.1 ± 0.2.

In one embodiment of this aspect, there is provided a crystal form of (R)-(2-(4-chlorophenyl)quinolin-4-yl)((S)-piperidin-2-yl)methanol (**S1**) in the form of an anhydrate (Form A1), wherein said crystal form has a powder X-ray diffraction pattern, as obtained with CuKα1-radiation, with characteristic peaks at diffraction angles (°2theta) of 10.4 ± 0.2, 19.1 ± 0.2, 12.7 ± 0.2, 14.7 ±0.2, 21.0 ±0.2 and 23.0 ±0.2.

In one embodiment of this aspect, there is provided a crystal form of (R)-(2-(4-chlorophenyl)quinolin-4-yl)((S)-piperidin-2-yl)methanol (**S1**) in the form of an anhydrate (Form A1), wherein said crystal form has a powder X-ray diffraction pattern, as obtained with CuKα1-radiation, with characteristic peaks at diffraction angles (°2theta) of 10.4 ± 0.2, 12.7 ± 0.2, 14.7 ± 0.2, 15.9 ± 0.2, 16.9 ± 0.2, 18.0 ± 0.2, 19.1 ± 0.2, 19.7 ± 0.2, 21.0 ± 0.2, 21.5 ± 0.2, 21.9 ± 0.2, 22.6 ± 0.2, 23.0 ± 0.2, 24.4 ± 0.2, 25.6 ± 0.2, 25.8 ± 0.2, 27.2 ± 0.2, 29.7 ± 0.2, 31.2 ± 0.2, 31.7 ± 0.2, 32.1 ± 0.2 and 32.8 ± 0.2.

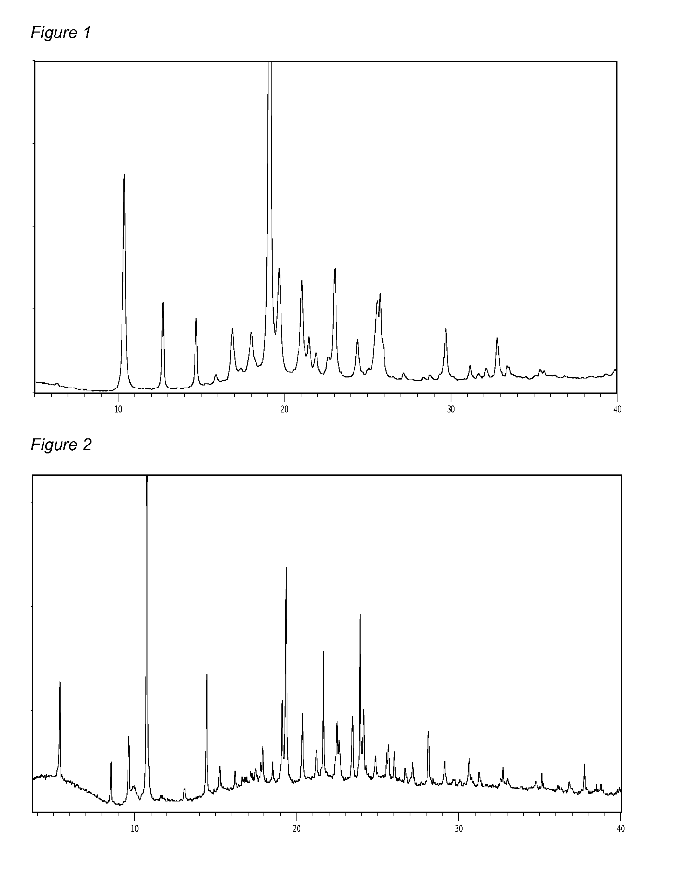

In one embodiment of this aspect, there is provided a crystal form of (R)-(2-(4-chlorophenyl)quinolin-4-yl)((S)-piperidin-2-yl)methanol (**S1**) in the form of an anhydrate (Form A1), wherein said crystal form has a powder X-ray diffraction pattern essentially as set out in Figure 1.

Another aspect of the invention provides a crystal form of (R)-(2-(4-chlorophenyl)quinolin-4-yl)((S)-piperidin-2-yl)methanol represented by compound **S1**: 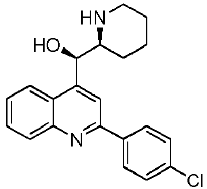 wherein said compound **S1** is in the form of a dihydrate (Form A2).

In one embodiment of this aspect, there is provided a crystal form of (R)-(2-(4-chlorophenyl)quinolin-4-yl)((S)-piperidin-2-yl)methanol (**S1**) in the form of a dihydrate (Form A2), wherein said crystal form has a powder X-ray diffraction pattern, as obtained with CuKα1-radiation, with characteristic peaks at diffraction angles (°2theta) of 5.4 ± 0.2, 10.8 ± 0.2 and 19.4 ± 0.2.

In one embodiment of this aspect, there is provided a crystal form of (R)-(2-(4-chlorophenyl)quinolin-4-yl)((S)-piperidin-2-yl)methanol (**S1**) in the form of a dihydrate (Form A2), wherein said crystal form has a powder X-ray diffraction pattern, as obtained with CuKα1-radiation, with characteristic peaks at diffraction angles (°2theta) of 5.4 ± 0.2, 10.8 ± 0.2, 19.4 ± 0.2, 14.4 ± 0.2, 21.6 ± 0.2 and 23.9 ± 0.2.

In one embodiment of this aspect, there is provided a crystal form of (R)-(2-(4-chlorophenyl)quinolin-4-yl)((S)-piperidin-2-yl)methanol (**S1**) in the form of a dihydrate (Form A2), wherein said crystal form has a powder X-ray diffraction pattern, as obtained with CuKα1-radiation, with characteristic peaks at diffraction angles (°2theta) of 5.4 ± 0.2, 8.5 ± 0.2, 9.6 ± 0.2, 10.8 ± 0.2, 14.4 ± 0.2, 15.2 ± 0.2, 17.9 ± 0.2, 18.5 ± 0.2, 19.1 ± 0.2, 19.4 ± 0.2, 20.3 ± 0.2, 21.2 ± 0.2, 21.6 ± 0.2, 22.5 ± 0.2, 22.7 ± 0.2, 23.4 ± 0.2, 23.9 ± 0.2, 24.1 ± 0.2, 24.9 ± 0.2, 26.0 ± 0.2, 26.7 ± 0.2, 27.1 ± 0.2, 28.1 ± 0.2, 29.1 ± 0.2, 30.7 ± 0.2 and 31.3 ± 0.2.

In one embodiment of this aspect, there is provided a crystal form of (R)-(2-(4-chlorophenyl)quinolin-4-yl)((S)-piperidin-2-yl)methanol (**S1**) in the form of a dihydrate (Form A2), wherein said crystal form has a powder X-ray diffraction pattern essentially as set out in Figure 2.

Another aspect of the invention provides a crystal form of (R)-(2-(4-chlorophenyl)quinolin-4-yl)((S)-piperidin-2-yl)methanol represented by compound **S1**: 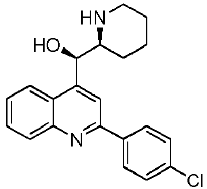 wherein said compound **S1** is in the form of a dichloroform solvate (Form A3).

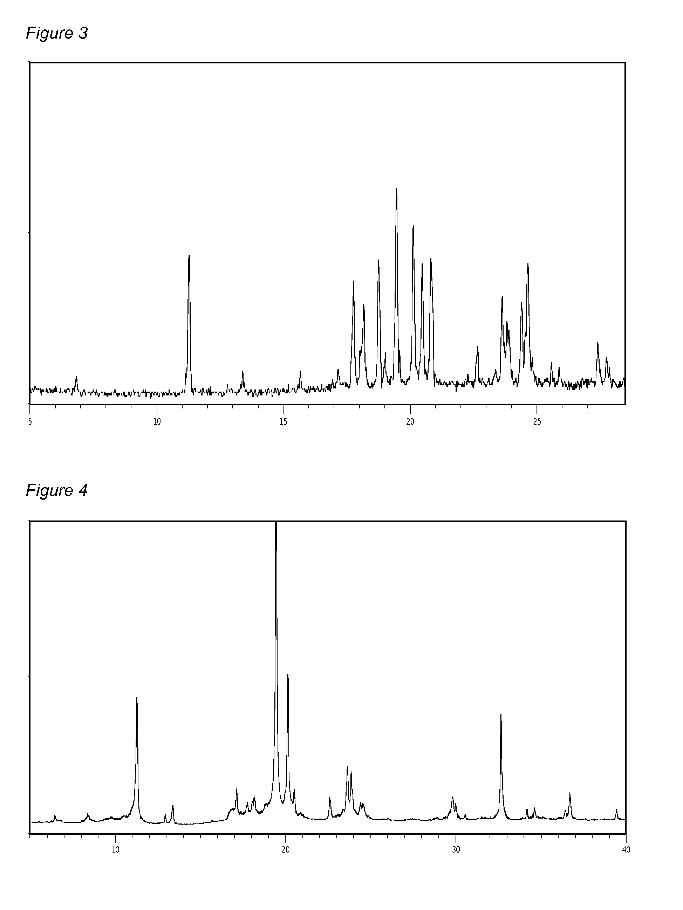

In one embodiment of this aspect, there is provided a crystal form of (R)-(2-(4-chlorophenyl)quinolin-4-yl)((S)-piperidin-2-yl)methanol (**S1**) in the form of a dichloroform solvate (Form A3), wherein said crystal form has a powder X-ray diffraction pattern essentially as set out in Figure 3.

Another aspect of the invention provides a crystal form of (R)-(2-(4-chlorophenyl)quinolin-4-yl)((S)-piperidin-2-yl)methanol represented by compound **S1**: 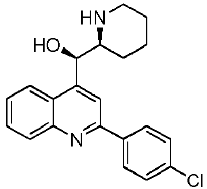 wherein said compound **S1** is in the form of a monochloroform solvate (Form A4).

In one embodiment of this aspect, there is provided a crystal form of (R)-(2-(4-chlorophenyl)quinolin-4-yl)((S)-piperidin-2-yl)methanol (**S1**) in the form of a monochloroform solvate (Form A4), wherein said crystal form has a powder X-ray diffraction pattern essentially as set out in Figure 4.

Another aspect of the invention provides a crystal form of (R)-(2-(4-chlorophenyl)quinolin-4-yl)((S)-piperidin-2-yl)methanol represented by compound **S1**: 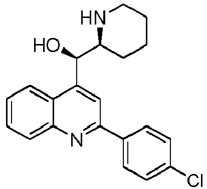 wherein said compound **S1** is in the form of a monohydrate (Form A5).

In one embodiment of this aspect, there is provided a crystal form of (R)-(2-(4-chlorophenyl)quinolin-4-yl)((S)-piperidin-2-yl)methanol (**S1**) in the form of a monohydrate (Form A5), wherein said crystal form has a powder X-ray diffraction pattern, as obtained with CuKα1-radiation, with characteristic peaks at diffraction angles (°2theta) of 9.2 ± 0.2, 10.3 ± 0.2 and 19.1 ± 0.2.

In one embodiment of this aspect, there is provided a crystal form of (R)-(2-(4-chlorophenyl)quinolin-4-yl)((S)-piperidin-2-yl)methanol (**S1**) in the form of a monohydrate (Form A5), wherein said crystal form has a powder X-ray diffraction pattern, as obtained with CuKα1-radiation, with characteristic peaks at diffraction angles (°2theta) of 9.2 ± 0.2, 10.3 ± 0.2, 19.1 ± 0.2, 8.3 ± 0.2, 16.6 ± 0.2 and 16.8 ± 0.2.

In one embodiment of this aspect, there is provided a crystal form of (R)-(2-(4-chlorophenyl)quinolin-4-yl)((S)-piperidin-2-yl)methanol (**S1**) in the form of a monohydrate (Form A5), wherein said crystal form has a powder X-ray diffraction pattern, as obtained with CuKα1-radiation, with characteristic peaks at diffraction angles (°2theta) of 5.7 ± 0.2, 7.5 ± 0.2, 8.3 ± 0.2, 9.2 ± 0.2, 10.3 ± 0.2, 12.7 ± 0.2, 12.9 ± 0.2, 14.7 ± 0.2, 15.2 ± 0.2, 15.8 ± 0.2, 16.6 ±0.2, 16.8 ± 0.2, 17.5 ± 0.2, 18.5 ± 0.2, 19.1 ±0.2, 19.7 ± 0.2, 21.0 ± 0.2, 23.0 ± 0.2, 24.6 ± 0.2 and 29.7 ± 0.2.

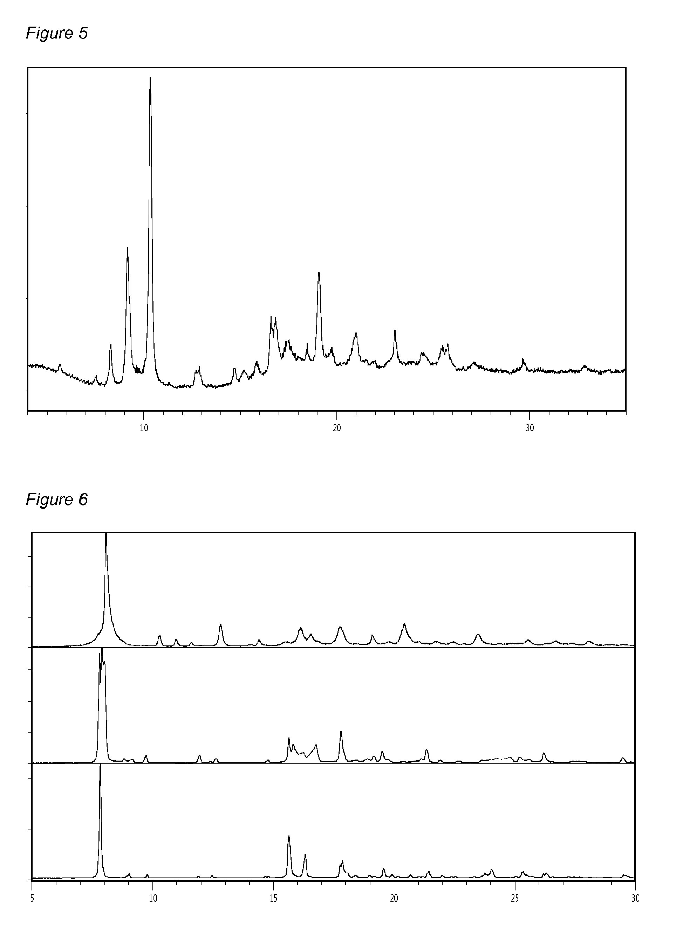

In one embodiment of this aspect, there is provided a crystal form of (R)-(2-(4-chlorophenyl)quinolin-4-yl)((S)-piperidin-2-yl)methanol (**S1**) in the form of a monohydrate (Form A5), wherein said crystal form has a powder X-ray diffraction pattern essentially as set out in Figure 5.

Another aspect of the invention provides a crystal form of (R)-(2-(4-chlorophenyl)quinolin-4-yl)((S)-piperidin-2-yl)methanol represented by compound **S1**: 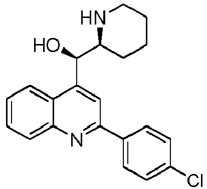 wherein said compound **S1** is in the form of a mono-DMF solvate (Form A6), a hemi-DMF solvate (Form A7) and a less DMF-containing form (Form A8).

In one embodiment of this aspect, there is provided crystal forms of (R)-(2-(4-chlorophenyl)quinolin-4-yl)((S)-piperidin-2-yl)methanol (**S1**) which are in the forms of DMF solvates (Form A6, A7 and A8), wherein said crystal forms have powder X-ray diffraction patterns essentially as set out in Figure 6.

Another aspect of the invention provides a crystal form of (R)-(2-(4-chlorophenyl)quinolin-4-yl)((S)-piperidin-2-yl)methanol represented by compound **S1**: 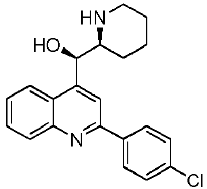 wherein said compound **S1** is in the form of an anhydrate (Form A10).

In one embodiment of this aspect, there is provided a crystal form of (R)-(2-(4-chlorophenyl)quinolin-4-yl)((S)-piperidin-2-yl)methanol (**S1**) which is in the form of an anhydrate (Form A10), wherein said crystal form has a powder X-ray diffraction pattern, as obtained with CuKα1-radiation, with characteristic peaks at diffraction angles (°2theta) of 10.4 ± 0.2 and 19.1 ± 0.2.

In one embodiment of this aspect, there is provided a crystal form of (R)-(2-(4-chlorophenyl)quinolin-4-yl)((S)-piperidin-2-yl)methanol (**S1**) in the form of an anhydrate (Form A10), wherein said crystal form has a powder X-ray diffraction pattern, as obtained with CuKα1-radiation, with characteristic peaks at diffraction angles (°2theta) of 10.4 ± 0.2, 19.1 ± 0.2, 10.1 ± 0.2, 14.7 ±0.2, 19.7 ± 0.2, 21.0 ± 0.2.

In one embodiment of this aspect, there is provided a crystal form of (R)-(2-(4-chlorophenyl)quinolin-4-yl)((S)-piperidin-2-yl)methanol (**S1**) in the form of an anhydrate (Form A10), wherein said crystal form has a powder X-ray diffraction pattern, as obtained with CuKα1-radiation, with characteristic peaks at diffraction angles (°2theta) of 5.9 ± 0.2, 8.9 ± 0.2, 10.1 ± 0.2, 10.4 ± 0.2, 12.7 ± 0.2, 14.7 ± 0.2, 15.6 ± 0.2, 15.9 ± 0.2, 16.9 ± 0.2, 17.4 ± 0.2, 18.1 ±0.2, 19.1 ±0.2, 19.7 ± 0.2, 21.0 ± 0.2, 21.5 ± 0.2, 21.9 ± 0.2, 22.6 ± 0.2, 23.0 ± 0.2, 24.1 ± 0.2, 24.4 ± 0.2, 25.5 ± 0.2, 25.8 ± 0.2, 27.1 ± 0.2, 27.8 ± 0.2, 28.4 ± 0.2, 28.8 ± 0.2, 29.7 ± 0.2, 31.2 ± 0.2, 31.7 ± 0.2, 32.1 ± 0.2, 32.8 ± 0.2 and 33.5 ± 0.2.

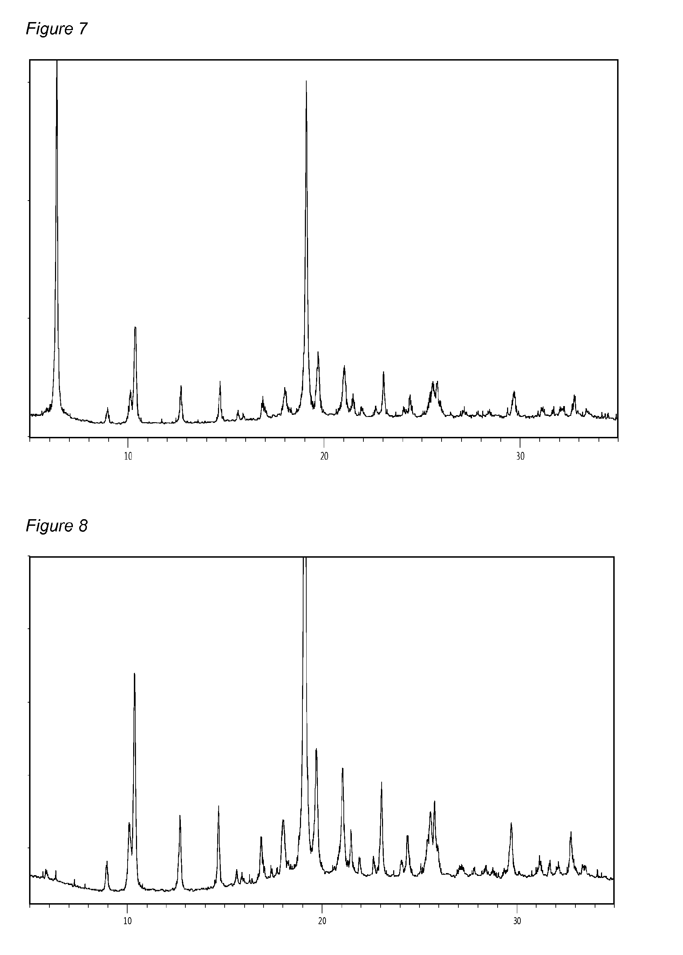

In one embodiment of this aspect, there is provided a crystal form of (R)-(2-(4-chlorophenyl)quinolin-4-yl)((S)-piperidin-2-yl)methanol (**S1**) in the form of an anhydrate (Form A10), wherein said crystal form has a powder X-ray diffraction pattern essentially as set out in Figure 8.

Another aspect of the invention provides a crystalline form of (R)-(2-(4-chlorophenyl)quinolin-4-yl)((S)-piperidin-2-yl)methanol dihydrochloride salt represented by compound **S1-2HCl**: 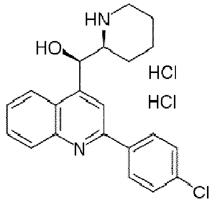 wherein said compound **S1-2HCl** is in the form of an anhydrate (Form B1).

In one embodiment of this aspect, there is provided a crystal form of (R)-(2-(4-chlorophenyl)quinolin-4-yl)((S)-piperidin-2-yl)methanol dihydrochloride salt (**S1-2HCl**) in the form of an anhydrate (Form B1), wherein said crystal form has a powder X-ray diffraction pattern, as obtained with CuKα1-radiation, with characteristic peaks at diffraction angles (°2theta) of 5.9 ± 0.2, 10.9 ± 0.2 and 26.1 ± 0.2.

In one embodiment of this aspect, there is provided a crystal form of (R)-(2-(4-chlorophenyl)quinolin-4-yl)((S)-piperidin-2-yl)methanol dihydrochloride salt (**S1-2HCl**) in the form of an anhydrate (Form B1), wherein said crystal form has a powder X-ray diffraction pattern, as obtained with CuKα1-radiation, with characteristic peaks at diffraction angles (°2theta) of 5.9 ± 0.2, 10.9 ± 0.2, 26.1 ± 0.2, 15.0 ± 0.2, 24.9 ± 0.2 and 25.1 ± 0.2.

In one embodiment of this aspect, there is provided a crystal form of (R)-(2-(4-chlorophenyl)quinolin-4-yl)((S)-piperidin-2-yl)methanol dihydrochloride salt (**S1-2HCl**) in the form of an anhydrate (Form B1), wherein said crystal form has a powder X-ray diffraction pattern, as obtained with CuKα1-radiation, with characteristic peaks at diffraction angles (°2theta) of 5.9 ± 0.2, 10.9 ± 0.2, 11.9 ± 0.2, 12.5 ± 0.2, 14.1 ± 0.2, 15.0 ± 0.2, 15.5 ± 0.2, 16.3 ± 0.2, 16.8 ± 0.2, 17.9 ± 0.2, 18.3 ± 0.2, 19.2 ± 0.2, 20.0 ± 0.2, 20.1 ± 0.2, 21.3 ± 0.2, 21.9 ± 0.2, 22.2 ± 0.2, 22.8 ± 0.2, 23.2 ± 0.2, 23.9 ± 0.2, 24.9 ± 0.2, 25.1 ± 0.2, 25.6 ± 0.2, 26.1 ± 0.2, 26.8 ± 0.2, 27.2 ± 0.2, 27.6 ± 0.2, 27.7 ± 0.2, 28.0 ± 0.2, 28.2 ± 0.2, 28.8 ± 0.2, 29.0 ± 0.2, 29.1 ± 0.2, 30.0 ± 0.2, 30.2 ± 0.2, 31.1 ± 0.2, 33.1 ± 0.2, 33.3 ± 0.2, 34.0 ± 0.2, 34.5 ± 0.2, 35.3 ± 0.2 and 35.5 ± 0.2.

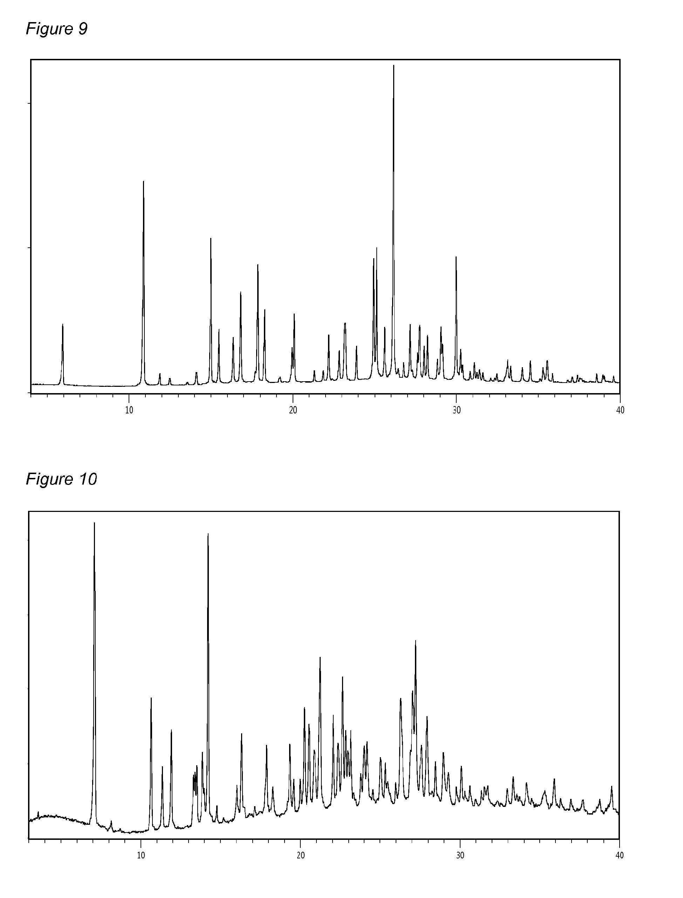

In one embodiment of this aspect, there is provided a crystal form of (R)-(2-(4-chlorophenyl)quinolin-4-yl)((S)-piperidin-2-yl)methanol dihydrochloride salt (**S1-2HCl**) in the form of an anhydrate (Form B1), wherein said crystal form has a powder X-ray diffraction pattern essentially as set out in Figure 9. Another aspect of the invention provides a crystal form of (R)-(2-(4-chlorophenyl)quinolin-4-yl)((S)-piperidin-2-yl)methanol dihydrochloride salt represented by compound **S1-2HCl**: 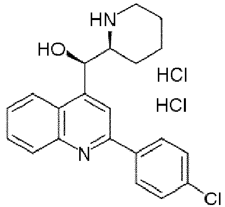 wherein said compound **S1-2HCl** is in the form of a dihydrate (Form B2).

In one embodiment of this aspect, there is provided a crystal form of (R)-(2-(4-chlorophenyl)quinolin-4-yl)((S)-piperidin-2-yl)methanol dihydrochloride salt (**S1-2HCl**) in the form of a dihydrate (Form B2), wherein said crystal form has a powder X-ray diffraction pattern, as obtained with CuKα1-radiation, with characteristic peaks at diffraction angles (°2theta) of 7.1 ± 0.2 and 14.2 ± 0.2.

In one embodiment of this aspect, there is provided a crystal form of (R)-(2-(4-chlorophenyl)quinolin-4-yl)((S)-piperidin-2-yl)methanol dihydrochloride salt (**S1-2HCl**) in the form of a dihydrate (Form B2), wherein said crystal form has a powder X-ray diffraction pattern, as obtained with CuKα1-radiation, with characteristic peaks at diffraction angles (°2theta) of 7.1 ± 0.2, 14.2 ± 0.2, 10.6 ± 0.2, 11.9 ± 0.2 and 21.2 ± 0.2.

In one embodiment of this aspect, there is provided a crystal form of (R)-(2-(4-chlorophenyl)quinolin-4-yl)((S)-piperidin-2-yl)methanol dihydrochloride salt (**S1-2HCl**) in the form of a dihydrate (Form B2), wherein said crystal form has a powder X-ray diffraction pattern, as obtained with CuKα1-radiation, with characteristic peaks at diffraction angles (°2theta) of 7.1 ± 0.2, 10.6 ± 0.2, 11.3 ± 0.2, 11.9 ± 0.2, 13.3 ± 0.2, 13.4 ± 0.2, 13.5 ± 0.2, 13.9 ± 0.2, 14.2 ± 0.2, 14.8 ± 0.2, 16.0 ± 0.2, 16.3 ± 0.2, 17.9 ± 0.2, 18.3 ± 0.2, 19.3 ± 0.2, 19.6 ± 0.2, 20.0 ± 0.2, 20.2 ± 0.2, 20.6 ± 0.2, 20.9 ± 0.2, 21.2 ± 0.2, 22.1 ± 0.2, 22.4 ± 0.2, 22.6 ± 0.2, 22.9 ± 0.2, 23.0 ± 0.2, 24.0 ± 0.2, 24.2 ± 0.2, 25.0 ± 0.2, 26.3 ± 0.2, 27.0 ± 0.2, 27.2 ± 0.2, 27.6 ± 0.2, 27.9 ± 0.2, 28.4 ± 0.2, 29.0 ± 0.2, 29.3 ± 0.2, 30.1 ± 0.2, 33.3 ± 0.2, 34.2 ± 0.2 and 35.9 ± 0.2.

In one embodiment of this aspect, there is provided a crystal form of (R)-(2-(4-chlorophenyl)quinolin-4-yl)((S)-piperidin-2-yl)methanol dihydrochloride salt (**S1-2HCl**) in the form of a dihydrate (Form B2), wherein said crystal form has a powder X-ray diffraction pattern essentially as set out in Figure 10.

A further aspect of the invention provides a pharmaceutical composition comprising any of the crystal forms A1, A2, A5, A10, B1 or B2 of compounds **S1** and **S1-2HCl** as described herein.

A further aspect of the invention provides an oral dosage form comprising any of the crystal forms A1, A2, A5, A10, B1 or B2 of compounds **S1** and **S1-2HCl** as described herein. For example, in one embodiment, said oral dosage form is a tablet, pill, dragee core or capsule.

There is provided a method for preparing a compound **S1** in crystal form A1, said method comprising dissolution of amorphous or crystalline **S1** in a substantially water free organic solvent, such as an alcohol, ketone, ester, ether or a carbohydrate, at a temperature between 25 - 150 °C, prefarably 25 - 100 °C, more preferably 25 - 75 °C, and then, if spontaneous crystallization does not occur, performing crystallization by cooling to room temperature, evaporation or the addition of an anti-solvent (such as n-heptane or water), or a combination of these methods, until a slurry of crystals in a satureted solution has been obtained. This slurry should then be stirred for a sufficient period of time to allow any metastable solvates to be transformed to form A1. The crystals are filtered off and are then dried slowly at room temperature, for example under reduced pressure, or at higher temperature, until the sample is dry.

Consequently, in one aspect of the invention, there is provided a method of preparing (R)-(2-(4-chlorophenyl)quinolin-4-yl)((S)-piperidin-2-yl)methanol (**S1**) in crystal form A1, said method comprising:
- dissolving amorphous or crystalline **S1** in a substantially water free organic solvent, at a temperature between 25 °C - 150 °C;
- performing crystallization by cooling to room temperature, evaporation or the addition of an anti-solvent, or a combination of these methods, until a slurry of crystals in a satureted solution has been obtained;
- stirring said slurry to allow metastable solvates to be transformed to form A1; and
- filtering and drying said A1 until the sample is dry.

There is provided a method for preparing a compound **S1** in crystal form A2, said method comprising dissolution of amorphous or crystalline **S1** in water or a mixture of 20 % (v/v), preferably 30 % (v/v), more prefarably 40 % (v/v), of water in an organic solvent, such as an alcohol, ketone, ester or ether at a temperature below 50 °C, preferably below 25 °C, more preferably below 5 °C and then, if spontaneous crystallization does not occur, performing crystallization by evaporation or the addition of an anti-solvent, or a combination of these, until a slurry of crystals in a satureted solution has been obtained. This slurry should then be stirred for a sufficient period of time to allow any metastable solvates to be transformed to form A2. The crystals are filtered off and are then dried slowly at a sufficiently low temperature that the structurally bound water molecules are not lost.

Consequently, in one aspect of the invention, there is provided a method of preparing (R)-(2-(4-chlorophenyl)quinolin-4-yl)((S)-piperidin-2-yl)methanol (**S1**) in crystal form A2, said method comprising:
- dissolving amorphous or crystalline **S1** in water or a mixture of water in an organic solvent, at a temperature below 50 °C;
- if spontaneous crystallization does not occur, performing crystallization by evaporation or the addition of an anti-solvent, or a combination of these, until a slurry of crystals in a satureted solution has been obtained;
- stirring said slurry to allow metastable solvates to be transformed to form A2; and
- filtering and drying said A2 until the sample is dry.

There is provided a method for preparing a compound **S1** in crystal form A5, said method comprising complete dissolution of amorphous or crystalline S1 in chloroform at reflux. The heating is then turned off and the vial with solution is opened so that the solution is allowed to evaporate and cool down, thus yielding crystals of form A3. The crystals are filtered off and are then dried slowly at room temperature, for example under reduced pressure, or at higher temperature, until all chloroform is removed. The dried material is then exposed to humid air with 30 - 40 %RH at room temperature until form A5 has formed.

Consquently, in one aspect of the invention, there is provided a method of preparing (R)-(2-(4-chlorophenyl)quinolin-4-yl)((S)-piperidin-2-yl)methanol (**S1**) in crystal form A5, said method comprising:
- complete dissolution of amorphous or crystalline **S1** in chloroform at reflux;
- the heating is turned off such that the solution is allowed to evaporate and cool down, yielding crystals of form A3;
- filtering and drying said A3 until all chloroform is removed; and
- the dried material is then exposing said dried A3 to humid air, at room temperature until form A5 is formed.

There is provided a method for preparing (R)-(2-(4-chlorophenyl)quinolin-4-yl)((S)-piperidin-2-yl)methanol 2HCl (**S1-2HCl**) in crystal form B1, said method comprising dissolution of amorphous or crystalline **S1-2HCl** in a substantially water free organic solvent, such as an alcohol, ketone, ester, ether or a carbohydrate, at a temperature between 25 - 150 °C, prefarably 25 - 100 °C, more preferably 25 - 75 °C, and then, if spontaneous crystallization does not occur, performing crystallization by cooling to room temperature, evaporation or the addition of an anti-solvent (such as n-heptane or water), or a combination of these methods, until a slurry of crystals in a saturated solution has been obtained. The crystals are filtered off and are then dried slowly at room temperature, for example under reduced pressure, or at higher temperature, until the sample is dry.

Consequently, in one aspect of the invention, there is provided a method of preparing (R)-(2-(4-chlorophenyl)quinolin-4-yl)((S)-piperidin-2-yl)methanol 2HCl (**S1-2HCl**) in crystal form, said method comprising:
- dissolving amorphous or crystalline **S1-2HCl** in a substantially water free organic solvent, at a temperature between 25 - 150 °C;
- if spontaneous crystallization does not occur, performing crystallization by cooling to room temperature, evaporation or the addition of an anti-solvent, or a combination of these methods, until a slurry of crystals in a saturated solution has been obtained; and
- filtering off and drying said B1 crystals, until the crystals are dry.

There is provided a method for preparing (R)-(2-(4-chlorophenyl)quinolin-4-yl)((S)-piperidin-2-yl)methanol 2HCl (**S1-2HCl**) in crystal form B2, said method comprising dissolution of amorphous or crystalline **S1-2HCl** in water or a mixture of 20 % (v/v), preferably 30 % (v/v), more prefarably 40 % (v/v), of water in an organic solvent, such as an alcohol, ketone, ester or ether at a temperature below 50 °C, preferably below 25 °C, more preferably below 5 °C and then, if spontaneous crystallization does not occur, performing crystallization by evaporation or the addition of an anti-solvent, or a combination of these, until a slurry of crystals in a satureted solution has been obtained. The crystals are filtered off and are then dried slowly at a sufficiently low temperature that the structurally bound water molecules are not lost.

Consequently, in one aspect of the invention, there is provided a method of preparing (R)-(2-(4-chlorophenyl)quinolin-4-yl)((S)-piperidin-2-yl)methanol 2HCl (**S1-2HCl**) in crystal form B2, said method comprising:
- dissolving amorphous or crystalline **S1-2HCl** in water or a mixture of water in an organic solvent, at a temperature below 50 °C;
- if spontaneous crystallization does not occur, performing crystallization by evaporation or the addition of an anti-solvent, or a combination of these, until a slurry of crystals in a saturated solution is obtained; and
- filtering off and drying said crystals slowly at a sufficiently low temperature such that structurally bound water molecules are not lost.

A further aspect of the invention provides a method of treating cancer in a mammal, the method comprising administering to the mammal a therapeutically effective amount of any of the crystal forms A1, A2, A5, A10, B1 or B2 of compound **S1** or compound **S1-2HCl** or any of the pharmaceutical compositions described herein.

A further aspect of the invention provides crystal forms of compound S1 or compound **S1-2HCl** as described herein, for use in the treatment of cancer.

A further aspect of the invention provides use of crystal forms of compound **S1** (or compound **S1-2HCl**) as described herein, in the preparation of a medicament useful in the treatment of cancer.

The cancer may be giloma, such as glioblastoma, including proneural, classical and mesenchymal glioblastoma.

The cancer may be a systemic form of cancer, such as pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/ lymphoid, cervical, pancreatic, hematological/lymphoid, pleura mesothelioma, renal, sarcoma, soft tissue, testicular or uterine body cancer.

It has surprisingly been identified that when crystallizing **S1** as crystal form A1, certain impurities can be reduced significantly, but not others. When crystallizing **S1-2HCl** as crystal form B1 it has, on the other hand been found that some of the impurities, not reduced when crystallized as **S1** crystal form A1, were reduced significantly. When aiming at crystallizing **S1,** crystal form A1, with as high a purity as possible, it is thus beneficial to first crystallize **S1-2HCl,** crystal form B1 and isolating it, before proceeding to dissolve this and recrystallizing it as **S1,** crystal form A1. Similarly, when aiming at crystallizing **S1-2HCl,** crystal form B1, with as high purity as possible, it is thus beneficial to first crystallize **S1,** crystal form A1 and isolating it, before proceeding to dissolve this and recrystallizing it as **S1-2HCl,** crystal form B1.

### Brief description of the drawings

Figure 1 shows a XRPD pattern of compound **S1,** Form A1, carried out on a PANalytical X'Pert PRO instrument, equipped with a Cu, long fine focus X-ray tube and a PIXcel detector. The x-axis shows 2-theta positions as obtained with CuK-alpha1 radiation.
Figure 2 shows a XRPD pattern of compound **S1,** Form A2, carried out on a PANalytical X'Pert PRO instrument, equipped with a Cu, long fine focus X-ray tube and a PIXcel detector. The x-axis shows 2-theta positions as obtained with CuK-alpha1 radiation.
Figure 3 shows a XRPD pattern of compound **S1,** Form A3, carried out on a PANalytical X'Pert PRO instrument, equipped with a Cu, long fine focus X-ray tube and a PIXcel detector. The x-axis shows 2-theta positions as obtained with CuK-alpha1 radiation.
Figure 4 shows a XRPD pattern of compound **S1,** Form A4, carried out on a PANalytical X'Pert PRO instrument, equipped with a Cu, long fine focus X-ray tube and a PIXcel detector. The x-axis shows 2-theta positions as obtained with CuK-alpha1 radiation.
Figure 5 shows a XRPD pattern of compound **S1,** Form A5, carried out on a PANalytical X'Pert PRO instrument, equipped with a Cu, long fine focus X-ray tube and a PIXcel detector. The x-axis shows 2-theta positions as obtained with CuK-alpha1 radiation.
Figure 6 shows XRPD patterns of compound **S1,** Form A6 (bottom), A7 (middle) and A8 (top), carried out on a PANalytical X'Pert PRO instrument, equipped with a Cu, long fine focus X-ray tube and a PIXcel detector. The x-axis shows 2-theta positions as obtained with CuK-alpha radiation.
Figure 7 shows a XRPD pattern of compound **S1,** Form A9, carried out on a PANalytical X'Pert PRO instrument, equipped with a Cu, long fine focus X-ray tube and a PIXcel detector. The x-axis shows 2-theta positions as obtained with CuK-alpha1 radiation.
Figure 8 shows a XRPD pattern of compound **S1**, Form A10, carried out on a PANalytical X'Pert PRO instrument, equipped with a Cu, long fine focus X-ray tube and a PIXcel detector. The x-axis shows 2-theta positions as obtained with CuK-alpha1 radiation.
Figure 9 shows a XRPD pattern of compound **S1-2HCl**, Form B1, carried out on a PANalytical X'Pert PRO instrument, equipped with a Cu, long fine focus

X-ray tube and a PIXcel detector. The x-axis shows 2-theta positions as obtained with CuK-alpha1 radiation.

Figure 10 shows a XRPD pattern of compound **S1-2HCl,** Form B2, carried out on a PANalytical X'Pert PRO instrument, equipped with a Cu, long fine focus X-ray tube and a PIXcel detector. The x-axis shows 2-theta positions as obtained with CuK-alpha1 radiation.

The foregoing and other aspects of the present invention will now be described in more detail with respect to the description and methodologies provided herein. It should be appreciated that the invention may be embodied in different forms and should not be construed as limited to the embodiments set forth herein. Rather, these embodiments are provided so that this disclosure will be thorough and complete, and will fully convey the scope of the invention to those skilled in the art.

The terminology used in the description of the invention herein is for the purpose of describing particular embodiments only and is not intended to be limiting of the invention. As used in the description of the embodiments of the invention, the singular forms "a," "an" and "the" are intended to include the plural forms as well, unless the context clearly indicates otherwise. Also, as used herein, "and/or" refers to and encompasses any and all possible combinations of one or more of the associated listed items. Furthermore, the term "about," as used herein when referring to a measurable value such as an amount of a compound, dose, time, temperature, and the like, is meant to encompass variations of 20%, 10%, 5%, 1%, 0.5%, or even 0.1 % of the specified amount. When a range is employed (*e.g.,* a range from x to y) it is it meant that the measurable value is a range from about x to about y, or any range therein, such as about x₁ to about y₁, etc. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. Unless otherwise defined, all terms, including technical and scientific terms used in the description, have the same meaning as commonly understood by one of ordinary skill in the art to which this invention belongs.

Any chiral center in a compound of the invention having a specified configuration is indicated as *R* or *S* using the well-known Cahn-Ingold-Prelog priority rules. Also, in any structural formula a chiral center having a specified configuration, (i.e. R or S) may be indicated using 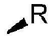 to indicate that the bond to R is directed out of the paper and towards the reader, and 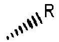 to indicate that the bond to R is directed out of the paper and away from the reader. Except, when otherwise indicated, e.g. by indication of (R) or (S) configuration at a given location, all stereoisomers of the compounds of the instant invention are contemplated, either in admixture or in pure or substantially pure form. Consequently, compounds of the invention may exist in enantiomeric or racemic or diastereomeric forms or as mixtures thereof. The processes for preparation can utilize racemates or enantiomers as starting materials.

The term "solvate" refers to a solid and crystalline complex of more or less variable stoichiometry, formed by a compound and a solvent.

The term "hydrate" refers to a solvate comprising the compound and a stoichiometric or non- stoichiometric amount of water.

The term "anhydrate" refers to a compound essentially free of water.

The term "2 HCl" "dihydrochloric acid" refers to a salt of a compound with essentially 2 formula units of HCl on each formula unit of compound.

The term "polymorphs" refer to crystalline materials of a compound which have different crystal structures and as a result of this different physical properties.

The term "crystal modification" or "crystal form" is the genereic term for ansolvates and solvates of a compound.

The term "crystalline" refers to any solid substance exhibiting three-dimensional order, which in contrast to an amorphous solid substance, gives a distinctive XRPD pattern with more or less sharp peaks.

The term "substantially pure" with reference to particular crystal forms, refers to that the crystal form includes less than 20 %, preferably less than 10 %, preferably less than 5 %, preferably less than 3 %, preferably less than 1 % by weight of any other crystalline or amorphous form of the compound.

The compounds, salts and prodrugs of the present invention can exist in several tautomeric forms, and such tautomeric forms are included within the scope of the present invention. Tautomers exist as mixtures of a tautomeric set in solution. In solid form, usually one tautomer predominates. Even though one tautomer may be described, the present invention includes all tautomers of the present compounds.

As used herein, the term "salt", such as a pharmaceutically acceptable salt, can include acid addition salts including hydrochlorides, hydrobromides, phosphates, sulphates, hydrogen sulphates, alkylsulphonates, arylsulphonates, acetates, benzoates, citrates, maleates, fumarates, succinates, lactates, and tartrates; alkali metal cations such as Na⁺, K⁺, Li⁺, alkali earth metal salts such as Mg²⁺ or Ca²⁺ and organic amine salts.

By "pharmaceutically acceptable salt" it is meant those salts which are, within the scope of sound medical judgment, suitable for use in contact with the tissues of humans and lower animals without undue toxicity, irritation, allergic response and the like, and are commensurate with a reasonable benefit/risk ratio.

For the purpose of the present invention "pharmaceutically acceptable" means that it is generally safe, non-toxic, and neither biologically nor otherwise undesirable and includes that it is acceptable for veterinary as well as human pharmaceutical use.

The term "effective amount" refers to an amount of a compound that confers a therapeutic effect on the treated patient. The effect may be objective (i.e. measurable by some test or marker) or subjective (i.e. the subject gives an indication of or feels an effect).

The term "DMF" refers to dimethyl formamide.

Pharmaceutically acceptable excipients for use in formulating a compound according to the invention as described and claimed herein, are for example, vehicles, adjuvants, carriers or diluents, which are well-known to those skilled in the art. Pharmaceutical excipients useful in formulating a compound as herein claimed and disclosed are found in e.g. Remington: The Science and Practice of Pharmacy, 19th ed., Mack Printing Company, Easton, Pennsylvania (1995).

The compounds of the invention can be administered by any suitable means, for example, orally, such as in the form of tablets, pills, dragees, aqueous or oily suspensions or solutions, elixirs, syrups, capsules, granules or powders; sublingually; buccally; parenterally, such as by e.g. subcutaneous, intravenous, intramuscular, or intrasternal injection or infusion techniques (e.g., as sterile injectable aqueous or non-aqueous solutions or suspensions). For parenteral administration, a parenterally acceptable aqueous or oily suspension, emulsion or solution is employed, which is pyrogen free and has requisite pH, isotonicity, osmolality and stability. Those skilled in the art are well able to prepare suitable formulations and numerous methods are described in the literature. A brief review of methods of drug delivery is also found in the scientific literature [eg. Langer, Science 249:1527-1533 (1990)].

Other examples of possible methods of administering the compounds of the invention are nasal administration including administration to the nasal membranes, such as by inhalation spray; or rectally such as in the form of suppositories; in dosage unit formulations containing non-toxic, pharmaceutically acceptable vehicles or diluents.

Preferably, the compounds of the present invention are parenterally administered in a way optimized for delivery to the brain of the treated subject. In one embodiment, the compounds are formulated for intraperitoneal administration. In one preferred embodiment, the compounds are formulated for intracerebroventricular administration.

The present compounds can also be administered in a form suitable for immediate release or extended release. Immediate release or extended release can be achieved by the use of suitable pharmaceutical compositions comprising the present compounds, or, particularly in the case of extended release, by the use of devices such as subcutaneous implants or osmotic pumps. The compounds of the invention can also be administered liposomally. The precise nature of the carrier or other material will depend on the route of administration and those skilled in the art are well able to prepare suitable solutions and numerous methods are described in the literature. Exemplary compositions for oral administration include suspensions which can contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which can contain, for example, microcrystalline cellulose, dicalcium phosphate, starch, magnesium stearate and/or lactose and/or other excipients, binders, extenders, disintegrants, diluents and lubricants such as those known in the art. The compounds of the invention can also be delivered through the oral cavity by sublingual and/or buccal administration. Molded tablets, compressed tablets or freeze-dried tablets are exemplary forms, which may be used. Exemplary compositions include those formulating the present compound(s) with fast dissolving diluents such as mannitol, lactose, sucrose and/or cyclodextrins. Also included in such formulations may be high molecular weight excipients such as celluloses (avicel) or polyethylene glycols (PEG). Such formulations can also include an excipient to aid mucosal adhesion such as hydroxy propyl cellulose (HPC), hydroxy propyl methyl cellulose (HPMC), sodium carboxy methyl cellulose (SCMC), maleic anhydride copolymer (e.g., Gantrez), and agents to control release such as polyacrylic copolymer (e.g. Carbopol 934). Lubricants, glidants, flavors, coloring agents and stabilizers may also be added for ease of fabrication and use.

Exemplary compositions for nasal aerosol or inhalation administration include solutions in saline, which can contain, for example, benzyl alcohol or other suitable preservatives, absorption promoters to enhance bioavailability, and/or other solubilizing or dispersing agents such as those known in the art.

Exemplary compositions for parenteral administration include injectable solutions, emulsions or suspensions which can contain, for example, suitable non-toxic, parenterally acceptable diluents or solvents, such as mannitol, 1,3-butanediol, water, Ringer's solution, an isotonic sodium chloride solution, oil or other suitable dispersing or wetting and suspending agents, including synthetic mono- or diglycerides, and fatty acids, including oleic acid, or Cremaphor.

Exemplary compositions for rectal administration include suppositories, which can contain, for example, a suitable non-irritating excipient, such as cocoa butter, synthetic glyceride esters or polyethylene glycols, which are solid at ordinary temperatures, but liquify and/or dissolve in the rectal cavity to release the drug.

The dose administered to a mammal, particularly a human, in the context of the present invention should be sufficient to effect a therapeutic response in the mammal over a reasonable time frame. One skilled in the art will recognize that dosage will depend upon a variety of factors including the potency of the specific compound, the age, condition and body weight of the patient, the extent of the condition being treated, recommendations of the treating physician, and the therapeutics or combination of therapeutics selected for administration, as well as the stage and severity of the disease. The dose will also be determined by the route (administration form), timing and frequency of administration. Oral dosages of the present invention, when used for the indicated effects, will range between about 0.01 mg per kg of body weight per day (mg/kg/day) to about 100 mg/kg/day, preferably 0.01 mg per kg of body weight per day (mg/kg/day) to 20 mg/kg/day, and most preferably 0.1 to 10 mg/kg/day, for adult humans. For oral administration, the compositions are preferably provided in the form of tablets or other forms of presentation provided in discrete units containing 0.5 to 1000 milligrams of the active ingredient for the symptomatic adjustment of the dosage to the patient to be treated, for example 0.5, 1.0, 2.5, 5.0, 10.0, 15.0, 25.0, 50.0, 100, 200, 400, 500, 600 and 800 mg.

Parenterally, especially intracerebroventricularly or intraperitoneally, the most preferred doses will range from about 0.001 to about 10 mg/kg/hour during a constant rate infusion. Advantageously, compounds of the present invention may be administered in single doses, e.g. once daily or more seldom, or in a total daily dosage administered in divided doses of two, three or four times daily.

Compounds of the present invention may also be used or administered in combination with at least one second therapeutic agent useful in the treatment of systemic cancer. The therapeutic agents may be in the same formulation or in separate formulations for administration simultaneously or sequentially. Compounds of the present invention may also be used in a combinational therapy or administered in combination with additional therapies, such as surgery and/or irradiation and/or other therapeutic strategies, including chemotherapies.

The term "treatment" as used throughout the specification and claims encompasses preventive therapy, palliative therapy or curative therapy. Thus, the term "treating" (or treatment) encompasses not only treating (or treatment of) a patient to relieve the patient of the signs and symptoms of the disease or condition, or to ameliorate the condition of the patient suffering from the disease or disorder, but also prophylactically treating an asymptomatic patient to prevent the onset or progression of the disease or condition. In one embodiment, the treatment is to relieve the patient of the signs and symptoms of the disease or condition, or to ameliorate the condition of the patient suffering from the disease or disorder or to prevent progression of the disease or condition.

As used herein, "treating," "treatment" or "treat" describes the management and care of a patient for the purpose of combating a disease, condition, or disorder and includes the administration of a compound of the present invention, to alleviate the symptoms or complications of a disease, condition or disorder, or to eliminate the disease, condition or disorder. The term "treat" can also include treatment of a cell *in vitro* or an animal model.

The term "patient(s)" include mammalian (including human) patient(s) (or "subject(s)"). As used herein, a "subject" is interchangeable with a "subject in need thereof", both of which refer to a subject having a disorder in which viral infection plays a part, or a subject having an increased risk of developing cancer relative to the population at large. A "subject" includes a mammal. The mammal can be *e.g.,* a human or appropriate non-human mammal, such as primate, mouse, rat, dog, cat, cow, horse, goat, camel, sheep or a pig. In one embodiment, the mammal is a human.

As used in the present disclosure, whether in a transitional phrase or in the body of a claim, the terms "comprise(s)" and "comprising" are to be interpreted as having an open-ended meaning. That is, the terms are to be interpreted synonymously with the phrases "having at least" or "including at least." When used in the context of a process the term "comprising" means that the process includes at least the recited steps, but may include additional steps. When used in the context of a molecule, compound, or composition, the term "comprising" means that the compound or composition includes at least the recited features or components, but may also include additional features or components.

It has been identified that the molecule (R)-(2-(4-chlorophenyl)quinolin-4-yl)((S)-piperidin-2-yl)methanol (**S1**) frequently crystallizes with solvent molecules included into its crystal structure. It has been ientified that this occurs with acetonitrile, dimethylformamide (DMF), ethanol, chloroform and water. Solvate formation is not a rare event in itself, but surprisingly several of the solvates identified are metastable and crystallize as hot solutions of **S1**, in organic solvents, cool down and evaporate. Typically, solvates are found at low temperatures and such solvates are usually thermodynamically stable in contact with the solvent. This is because solvent molecules are volatile and lower temperatures lower the solvent vapor pressure and thus stabilizes solvated crystal structures. The mono-DMF solvate and the dihydrates, form A2 of **S1** and form B2 of **S1-2HCl** are examples of thermodynamically stable solvates. The other solvates are metastable as proven by the fact that when **S1** is slurried in these solvents for a period of a week, they do not occur. Typically, solvates of a pharmaceutically active molecule and an organic solvent cannot be used in pharmaceutical formulations since most organic solvents are generally toxic. However, it has been shown that several of these solvates can be desolvated to provide useful ansolvates, e.g. form A10, or even a monohydrate (form A5). Potentially this is also the case for form A8.

It is noted that such metastable solvates are rather unstable in contact with air and loses solvent rapidly. However, by using careful experimental and analytical procedures, it has been possible to obtain both correct XRPD-data and the crystal structures.

All percentages and ratios used herein, unless otherwise indicated, are by weight. Other features and advantages of the present invention are apparent from the different examples. The provided examples illustrate different components and methodology useful in practicing the present invention. The examples do not limit the claimed invention. Based on the present disclosure the skilled artisan can identify and employ other components and methodology useful for practicing the present invention.

### Experimental methods

### X-Ray Powder Diffraction (XRPD) analysis

Dry samples were lightly ground in an agate mortar, if needed, and were then smeared out on a sample holder. Slurry samples were added to the sample holder as wet and were analyzed both wet and dry. XRPD data were collected on a cut Silicon Zero Background Holder (ZBH) or on a Porous Alumina Filter Sample Holder, using a PANalytical X'Pert Pro diffractometer, equipped with aPIXcel detector. The sample was spun during analysis and Cu-radiation was used. The following experimental settings were used:
Tube tension and current: 40 kV, 50 mA
Wavelength alpha1 (CuKα1): 1.5406 A
Wavelength alpha2 (CuKα2): 1.5444 A
Wavelength alpha1 and alpha2 mean (CuKα): 1.5418 A
Start angle [2 theta]: 1 - 4 °
End angle [2 theta]: 30 - 50 °
Analysis time: 45 or 76 s ("1 min scan"), 434 s ("7 min scan"), 577 s ("10 min scan"), 1020 s ("17 min scan"), 1256 s ("21 min scan"), 3720 s ("1 h scan"), 8007 s ("2.5 h scan")

Unless indicated otherwise, when calculating the peak positions from the XRPD-data, the data was first stripped from the contribution from CuKα2 and was then corrected against an internal standard (Al₂O₃).

It is known in the art that an X-ray powder diffraction pattern may be obtained having one or more measurement errors depending on measurement conditions (such as equipment, sample preparation or machine used). In particular, it is generally known that intensities in an XRPD pattern may fluctuate depending on measurement conditions and sample preparation. For example, persons skilled in the art of XRPD will realise that the relative intensities of peaks may vary according to the orientation of the sample under the test and on the type and setting of the instrument used. The skilled person will also realise that the position of reflections can be affected by the precise height at which the sample sits in the diffractometer and the zero calibration of the diffractometer. The surface planarity of the sample may also have a small effect. Hence a person skilled in the art will appreciate that the diffraction pattern presented herein is not to be construed as absolute and any crystalline form that provides a powder diffraction pattern substantially identical to those disclosed herein fall within the scope of the present disclosure (for further information, see R. Jenkins and R.L. Snyder, "Introduction to X-ray powder diffractometry", John Wiley & Sons, 1996),

### Thermogravimetric analysis (TGA)

Approximately 1- 5 mg of sample was added to a tared Platinum or Aluminium cup which was then placed in the weighting position of a Mettler Toledo TGA/SDTA 851. The furnace was closed and the starting weight of the sample was recorded. The heating program was then started. The sample was heated at a rate of 5 - 10 °C/min, starting at 25°C and ending at 60 - 300°C, depending on where a constant weight could be attained. The sample was purged with dry nitrogen gas during analysis. Some samples, after being heated to a higher temperature, were kept at the higher temperature for 30 - 90 minutes.

### Examples

### Example 1.

### Stereoselective synthesis of (R)-(2-(4-chlorophenyl)quinolin-4-yl)((S)-piperidin-2-yl)methanol (S1)

A stereoselective synthesis of **S1** was designed based on a modification of León (León, B., et al (2013). Organic Letters, 15(6), 1234-7), according to following Scheme 2.

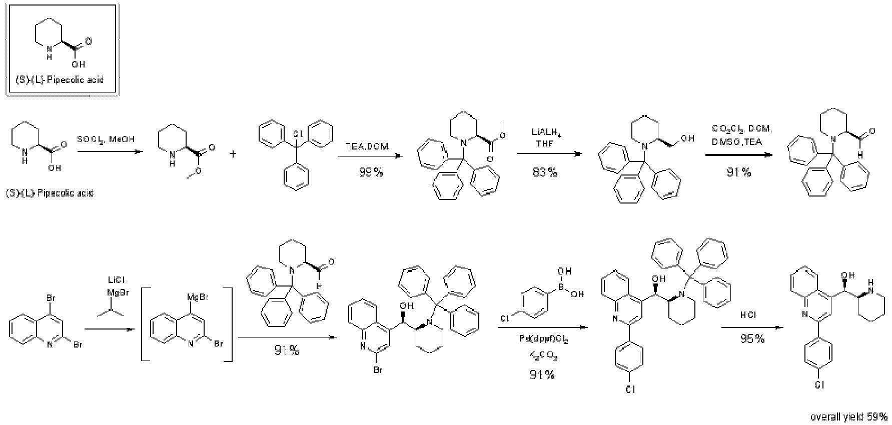

Briefly, tritylation of methylated (S)-L-Pipecolic acid afforded the possibility to generate a chiral piperidine carbaldehyde material suitable for face-selective addition by the Grignard reagent generated from 2,4-dibromoquinoline. The single isolated R,S isomer was then subject to Suzuki coupling of the appropriate 4-chlorophenylboronic acid, which after concomitant deprotection of the trityl group yields the desired (R)-[2-(4-chlorophenyl)quinolin-4-yl](2S)-piperidin-2-ylmethanol (**S1**).

### Methyl (2S)-piperidine-2-carboxylate

(S)-(L)-Pipecolic acid (6.1 g, 47.2 mmol) was added to methanol (47.2 mL) under N₂. To this solution thionyl chloride (6.9 mL, 94.3 mmol) was slowly added at -10 °C. The reaction mixture was allowed to warm to rt and was stirred for 18 hours. Reaction mixture was evaporated and co-evaporated with toluene and dried under vacuum. The crude was used in next step.

### Methyl (2S)-1-(triphenylmethyl)piperidine-2-carboxylate

Methyl (2S)-piperidine-2-carboxylate (6.8 g, 47.2 mmol) was dissolved in CH₂Cl₂ (57 mL), then Et₃N (19.7 mL, 141.5 mmol) was added. To this solution was added trityl chloride (13.1 g, 47.2 mmol) and the reaction mixture was stirred for 18 h at rt. The reaction was hydrolyzed with NH₄Cl/28% NH₃ (40 mL, 2:1). The solution was partitioned between Et₂O (80 mL) and H₂O (80 mL). The layers were separated and the aqueous layer was extracted with Et₂O (3 x 50 mL). The combined organic layers were dried with Na₂SO₄, filtered, and concentrated *in vacuo.* The residue was purified by flash chromatography (1:2:97, Et₃N:EtOAc:Heptane) to give title compound (18.2 g, 47.2 mmol, 99% yield) as a white foam. MS ESI+ m/z 144 [M+H-Tr]+.

### [(2S)-1-(triphenylmethyl)piperidin-2-yl]methanol

To an oven dried 3-neck flask equipped with a stir bar (N2) and condenser was added THF (55 mL). To this solution was added LiAIH4 (3.2 g, 86.2 mmol) and was allowed to stir to form a suspension. To this suspension was added methyl (2S)-1-(triphenylmethyl)piperidine-2-carboxylate (22.1 g, 57.4 mmol). The reaction solution was allowed to stir for 3h at rt (became thick suspension after 30 min and 10 ml THF was added). The reaction mixture was then cautiously quenched with NaOH (11 mL, 1.0 M), and H₂O (22 mL). The solution became visibly thicker and more difficult to stir. MgSO₄ was then added and the solution was passed through a pad of celite with 300 mL of dichloromethane. This was then concentrated *in vacuo.* The residue was purified by flash chromatography (1:1:98, Et3N:MeOH: CH₂Cl₂) to title compound (17 g, 47.6 mmol, 83% yield) as a white foam. MS ESI+ m/z 116 [M+H-Tr]+. ¹H NMR (400 MHz, DMSO-d₆) δ 7.42 (d, *J* = 7.58 Hz, 6H), 7.28 (t, *J* = 7.74 Hz, 6H), 7.15 (t, *J* = 7.70 Hz, 3H), 3.93 - 3.99 (m, 1 H), 3.11 - 3.24 (m, 2H), 2.74 - 2.84 (m, 1 H), 2.26 - 2.38 (m, 2H), 1.60 - 1.76 (m, 2H), 1.43 - 1.59 (m, 2H), 1.35 (br. s., 2H). ¹³C NMR (101 MHz, DMSO-d₆) δ 144.9, 128.8, 127.7, 125.8, 77.2, 59.7, 56.1, 54.5, 42.1, 25.1, 18.6

### (2S)-1-(triphenylmethyl)piperidine-2-carbaldehyde

To an oven dried flask equipped with a stir bar (under N₂) was added CH₂Cl₂ (57 mL) and was then taken to -78 °C. To this solution was slowly added (COCl₂) (6.1 mL, 71.3 mmol). Next a solution of DMSO (8.4 mL, 118.9 mmol) in CH₂Cl₂ (33 mL) was added dropwise. This was allowed to stir for 10 min and a solution of [(2S)-1-(triphenylmethyl)piperidin-2-yl]methanol (17 g, 47.6 mmol) in CH₂Cl₂ (43 mL) was then added. The suspension was allowed to stir for 2 h and then Et₃N (26.5 mL, 190.2 mmol) was added and allowed to stir for an additional 3 h. The -78 °C bath was then removed and NH₄Cl/28% NH₃ (100 mL, 2:1) was added and the solution was partitioned between CH₂Cl₂ (60 mL) and H₂O (60 mL). The layers were separated and the aqueous layer was extracted with CH₂Cl₂ (3 x 70 mL). The combined organic layers were dried with Na₂SO₄, filtered, and concentrated *in vacuo.* The residue was purified by flash chromatography (1:9:90, Et3N:EtOAc:Heptane) to afford title compound (1 g, 91%) as a white solid. MS ESI+ m/z 114 [M+H-Tr]+.¹H NMR (400 MHz, DMSO-d₆) δ 8.43 (s, 1 H), 7.42 (d, *J* = 7.42 Hz, 6H), 7.32 (t, *J* = 7.74 Hz, 6H), 7.16 - 7.24 (m, 3H), 3.55 (d, *J* = 1.74 Hz, 1 H), 3.12 - 3.21 (m, 1 H), 2.77 (s, 1 H), 1.86 - 2.00 (m, 1 H), 1.74 - 1.83 (m, 1 H), 1.62 - 1.74 (m, 1 H), 1.57 (d, *J* = 11.22 Hz, 2H), 1.01 - 1.18 (m, 1 H). ¹³C NMR (101 MHz, DMSO-d₆) δ 208.9, 143.4, 128.6, 128.2, 126.3, 76.9, 62.0, 43.1, 28.9, 25.2, 20.7

### (S)-(2-bromoquinolin-4-yl)[(2R)-1-(triphenylmethyl)piperidin-2-yl]methanol

2,4-dibromoquinoline (5.0 g, 17.6 mmol, 1.1 equiv.) was dissolved in dry tetrahydrofuran (42 mL). i-PrMgCl LiCl complex 1.3 M solution in tetrahydrofuran (16.0 mL, 20.8 mmol, 1.3 equiv.) was added slowly, dropwise, at -5 - 0°C. The reaction mixture was stirred at rt for 20 min. (2R)-1-(triphenylmethyl)piperidine-2-carbaldehyde (5.7 g, 16.0 mmol, 1.0 equiv.) was added in one portion at 0°C and reaction was allowed to reach room temperature and the reaction mixture was stirred at rt for 5h. After the reaction was completed NH₄Cl (sat.)/NH₃(28%) (30 ml) solution was added and the mixture was extracted with DCM (3 x 20 mL). The organic phase was separated and washed with brine (20 mL) The solution was dried over Na₂SO₄, filtered and then evaporated in vacuo. The resultant was chromatographed on silica gel eluting with TEA:ethyl acetate:heptane (1:10:90). Crude was dissolved in TEA/ethyl acetate/heptane (1:10:90) and EtOAc was added to get solution. SiO₂ was added to the solution and the mixture was evaporated to dryness and was added to SiO₂ column (d=7 cm x h=10 cm) eluting with TEA/ethyl acetate/heptane (1:10:90) to (1:10:40). Fractions were collected and dried under vacuum to give title compound (8.2 g, 14.5 mmol, 91% yield) as a white solid. Exact mass for C₃₄H₃₂BrN₂O [M+H]+ requires m/z 563.1698, HPLC-MS (API-ES) (ACE C8 10-90% MeCN 1.5 min (0.1% TFA pH 2) (API-ES) C₁₅H₁₈CIN₂O [M+H]+ requires m/z 321.0602 found m/z 321, (Trityl-group was removed in acidic condition). ¹H NMR (400 MHz, DMSO-d₆) δ 7.92 - 7.98 (m, 1 H), 7.81 (s, 1 H), 7.70 - 7.76 (m, 1 H), 7.38 (d, *J* = 6.95 Hz, 6H), 7.24 (s, 1 H), 7.12 - 7.22 (m, 9H), 6.01 (d, *J* = 4.74 Hz, 1 H), 5.72 - 5.80 (m, 1 H), 3.68 - 3.76 (m, 1 H), 3.60 - 3.68 (m, 1 H), 2.95 - 3.05 (m, 1 H), 1.65 - 1.83 (m, 1 H), 1.20 - 1.31 (m, 1 H), 0.95 - 1.13 (m, 4H), 0.07 - 0.28 (m, 1 H). ¹³C NMR (101 MHz, DMSO-d₆) δ 154.9, 148.1, 144.8, 141.6, 130.3, 129.8, 128.7, 127.6, 126.6, 126.0, 124.8, 124.7, 123.8, 78.4, 73.6, 55.6, 31.1, 22.6, 18.5, 14.0

### (R)-[2-(4-chlorophenyl)quinolin-4-yl][(2S)-1-(triphenylmethyl)piperidin-2-yl]methanol

(R)-(2-bromoquinolin-4-yl)[(2S)-1-(triphenylmethyl)piperidin-2-yl]methanol (7.9 g, 14.0 mmol, 1.0 equiv.) and 4-chlorophenylboronic acid (2.3 g, 14.7 mmol, 1.05 equiv.) were dissolved in 2-MeTHF (5.5 mL) under N₂, PdCl₂(dppf) (0.23 g, 0.28 mmol, 0.02 equiv.) and 2M K₂CO₃ (28.0 mL, 55.8 mmol, 4 equiv.) were added under nitrogen atmosphere and the reaction was heated at 80 °C for 5h, conversion followed by HPLC. Phases was separated and organic phase was evaporated to dryness and dissolved in EtOAc (7 mL) and heptane (10 mL) was added. The resulting solution was filtered through silica gel eluting with triethylamine/ethyl acetate/heptane (1:20:80). First 400 mL containing product was evaporated to give title compound (7.6 g, 12.7 mmol, 91% yield). HPLC-MS (API-ES) Exact mass for C₄₀H₃₅CIN₂O [M+H]+ requires m/z 594.2437. HPLC-MS (API-ES) (ACE C8 10-90% MeCN 1.5 min (0.1% TFA pH 2) (API-ES) C₂₁H₂₁CIN₂O [M+H]+ requires m/z 353.1421, found m/z 353, (Trityl-group was removed in acidic condition). ¹H NMR (400 MHz, DMSO-d₆) δ 8.29 - 8.36 (m, 3H), 8.08 (dd, *J* = 0.95, 8.53 Hz, 1 H), 7.71 (ddd, *J* = 1.11, 6.95, 8.37 Hz, 1 H), 7.64 - 7.68 (m, 2H), 7.46 (br. d, *J* = 8.50 Hz, 1 H), 7.30 - 7.38 (m, 6H), 7.21 (ddd, *J* = 1.26, 6.95, 8.37 Hz, 1 H), 7.10 (d, *J* = 2.21 Hz, 8H), 5.94 (d, *J* = 4.74 Hz, 1 H), 5.85 (t, *J* = 5.29 Hz, 1 H), 3.71 - 3.82 (m, 2H), 2.95 - 3.05 (m, 1 H), 1.73 - 1.88 (m, 1 H), 1.20 - 1.30 (m, 2H), 1.08 - 1.15 (m, 1 H), 0.97 - 1.07 (m, 2H), 0.08 - 0.25 (m, 1 H)

### (R)-[2-(4-chlorophenyl)quinolin-4-yl](2S)-piperidin-2-ylmethanol dihydrochloride (S1)

(R)-[2-(4-chlorophenyl)quinolin-4-yl][(2S)-1-(triphenylmethyl)piperidin-2-yl]methanol (7.6 g, 12.7 mmol) was dissolved in Et₂O (114 mL) followed by addition of 3M HCl (197 mL). After stirring at room temperature for 4 h, the solution was partitioned between Et₂O (70 mL) and H₂O (400 mL). The aqueous layer was extracted with Et₂O (3 x 80 mL). The aqueous layer was then basified with 6M NaOH (50 mL gave pH 12-13), and then was extracted with CH₂Cl₂ (200 mL). The CH₂Cl₂ layer was dried with Na₂SO₄, filtered, and concentrated in vacuo. Product was solved in CH₂Cl₂ 50 ml and 1 M HCl in Et₂O (12 ml) was added droppwise at 0 °C under stirring, precipitate was collected and dried under vacuum to give title compound dihydrochloride salt (5.1 g, 12.0 mmol, 95% yield) as a white solid. HPLC-MS (API-ES) Exact mass for C₂₁H₂₁CIN₂O [M+H]+ requires m/z 353.1421, found m/z 353. 1H NMR (400 MHz, DMSO-d₆) δ 10.45 (d, *J* = 9.79 Hz, 1 H), 8.60 (d, *J* = 8.37 Hz, 1 H), 8.47 (q, *J* = 10.37 Hz, 1 H), 8.25 - 8.31 (m, 3H), 8.22 (s, 1 H), 7.87 - 7.94 (m, 1 H), 7.73 (ddd, *J* = 1.11, 7.03, 8.29 Hz, 1 H), 7.66 - 7.70 (m, 2H), 6.07 (d, *J* = 1.90 Hz, 1 H), 3.35 - 3.46 (m, 1 H), 3.24 (d, *J* = 11.69 Hz, 1 H), 2.89 - 3.03 (m, 1 H), 1.50 - 1.78 (m, 3H), 1.16 - 1.34 (m, 1 H).

### Example 2.

### Preparation of amorphous free base from amorphous HCl-salt.

To a sample of amorphous **S1-2HCl** (306.55 mg) Sodium Hydroxide (5 mL 1 M) was added at room temperature (about 21 °C) under agitation by a magnetic stirrer in a small round bottom flask. Methyl tert-butyl ether was added (5mL) and the sample was shaken. More Sodium Hydroxide (1 mL) was added and after some minutes two clear liquid phases appeared. The upper phase (organic) was separated from the water phase in a separation funnel. The water phase was washed with 6 ml Methyl tert-butyl ether, which then was separated and added to the previously isolated Methyl tert-butyl ether phase. Then the total Methyl tert-butyl ether portion was evaporated in a rotary evaporator at vacuum and 40°C to a white solid product, which was further dried in a drying oven at reduced pressure and at 40°C for 1.5 hours to a visually (polarized light microscopy) amorphous powder of **S1** (isolated amount 274.44 mg).

### Example 3.

### Preparation of S1 form A1 from amorphous material.

Amorphous **S1** (20 mg) from example 2 was mixed with 0.10 mL of acetone at room temperature (about 21 °C). The slurry sample was stirred for one day. The sample was then analyzed by XRPD which showed that form A1 (as in Figure 1) had been obtained.

### Example 4.

### Preparation of S1 form A1 from S1-2HCl.

**S1-2HCl** (26.04g) was suspended in water (260 ml) and tert-butyl methyl ether (160 ml). The pH was adjusted to 14 with 45% sodium hydroxide. The mixture was warmed to 55°C and filtered. The resulting two phase liquid system was filtered, the phases separated and the aqueous phase was discarded. The organic phase was concentrated under reduced pressure. The residue was dissolved in isopropyl acetate (130 ml) and the mixture was warmed to 88°C and the remaining tert-butyl methyl ether was distilled off. The resulting solution was adjusted to 130 ml volume with fresh isopropyl acetate. The solution was cooled to 20°C during 4 hours. To the resulting thick slurry was added isopropyl acetate (10 ml) and the mixture was warmed to 88°C. Isopropyl acetate (20 ml) was added to the slurry which was cooled to 20°C during 2 hours. The solids were isolated by filtration, washed with cold isopropyl acetate (45 ml) and were dried under reduced pressure at 40°C giving **S1** (13.04 g). XRPD-analysis showed that this material was form A1 (as in Figure 1). No significant weight losses were seen in a TGA-analysis, thus indicationg that form A1 is an ansolvate.

### Example 5.

### Preparation of S1 forms A9 and A10 from amorphous S1.

Amorphous **S1** (20 mg) from example 1 was mixed with 0.10 mL of ethanol at room temperature (about 21 °C). The slurry sample was stirred for one day. The sample was then analyzed by XRPD which showed that a new form had crystallized (see Figure 7). The large peak at 6.4 ° in 2-theta however gradually lost intensity as the sample was left in open air and eventually disappeared completely. This leads to the conclusion that this is an ethanol solvate. The dry sample was analyzed with TGA and contained no significant amounts of solvent. This dried material is thus an ansolvate, form A10 (as in Figure 8). The experiment could not be reproduced.

### Example 6.

### Preparation of S1 form A1 from amorphous material.

In an attempt to reproduce example 5, 50 mg of amorphous **S1** was added to a vial. Then ethanol was added (0.25 ml) and the substance was partly dissolved. After agitating the slurry for approximately 10 minutes at room temperature (about 21 °C) a free flowing slurry started to form. The slurry was analyzed by XRPD showing that form A1 (as in Figure 1) was obtained. No sign of form A9 (as in Figure 7) was seen.

### Example 7.

### Preparation of S1 form A1 by slurry in acetone.

22 mg of **S1**, form A1 was slurried in 0.63 ml acetone at 22 °C during 7 days at room temperature (about 21 °C). The sample was analyzed with XRPD showing that form A1 (as in Figure 1) was still present.

### Example 8.

### Preparation of S1 form A1 by anti-solvent crystallization.

31 mg of **S1**, form A1 was completely dissolved in 0.6 ml ethanol at room temperature (about 21 °C). Water (1.8 ml) was added as anti-solvent. Crystallization started immediately and a sample was taken for XRPD-analysis after 5 minutes. The sample had crystallized to form A1 (as in Figure 1).

### Example 9.

### Preparation of S1 form A1 by anti-solvent crystallisation.

22mg of **S1**, form A1 was completely dissolved in 1.1 ml toluene at room temperature (about 21 °C). Heptane (1.0 ml) was added as anti-solvent. Crystallization started immediately and a sample was taken for XRPD-analysis after 10 minutes, which showed that the sample had crystallized to form A1 (as in Figure 1).

### Example 10.

### Preparation of S1 form A1 by cooling crystallization

21 mg of **S1**, form A1 was completely dissolved in acetonitrile (2 ml) at 65°C. The solution was then cooled down to 22 °C by natural cooling. The resulting slurry was analyzed by XRPD after 10 minutes. The analysis showed that form A1 (as in **Figure 1**) had been obtained.

### Example 11.

### Preparation of S1 form A1 by evaporative crystallization

20 mg of **S1**, form A1 was completely dissolved in acetone (1 ml) at room temperature (about 21 °C). The solution was allowed to slowly evaporate to dryness. The resulting product was analyzed by XRPD showing that form A1 (as in Figure 1) had been obtained.

### Example 12.

### Preparation of S1 form A2

18.7 mg of **S1**, form A1 was slurried in 0.752 g of a mixture of 1-propanol and water (50:50 % (v/v)) at 5 °C during 8 days. The sample was analyzed by XRPD showing that a new form had been obtained (Form A2 as in Figure 2). Two TGA-analyses of this sample, performed after different times of storage, showed that Form A2 contained 7.8 - 11.4 %w/w of a solvent.

### Example 13.

### Preparation of a mixture of forms A1 and A2 of S1

19.9 mg **of S1,** form A1 was slurried in water (0.605 g solvent) at 5 °C during 8 days. The sample was analyzed by XRPD showing that a mixture of form A1 (as in Figure 1) and form A2 (as in Figure 2) had been obtained. This, together with the information in example 12, shows that form A2 is a hydrate. The TGA-data from example 12 shows that it contains 1.5 - 2.5 moles of water per mole of **S1.** Form A2 has therefore been designated as a dihydrate.

### Example 14.

### Preparation of chloroform solvates of S1 (forms A3 and A4)

26.4 mg of **S1** was completely dissolved in chloroform by heating in a 0.5 ml vial. The heating was turned off and crystallization started after approximately 1 h. The sample was analyzed by single crystal XRD and XRPD showing that a dichloroform solvate, form A3 (a in Figure 3), had been obtained. During drying with simultaneous XRPD-analysis the dichloroform solvate was shown to transforms to a new crystalline phase, form A4 (as in Figure 4). This sample was analyzed with TGA and was shown to contain 22 % (w/w) of chloroform. Form A4 is thus close to a monochloroform solvate (theoretical value 25.2 % (w/w)).

### Example 15.

### Preparation of monohydrate form A5 of S1 by drying and rehydration of S1 form A4.

A sample of **S1,** form A4 was analyzed with TGA, by heating it to 70 °C with 10 °C/minute and then holding it there for 90 minutes The sample lost 18.1 % w/w and was then analyzed with XRPD, which showed that a new form (form A5 as in Figure 5) had been obtained. The sample was then re-analyzed with TGA and then once again with XRPD. The sample had now lost 5.2 % (w/w) but the XRPD-data was still that of form A5 (as in Figure 5). This shows that the dried sample had taken up moisture from the surrounding air and formed a monohydrate (theoretical water content 4.9 % (w/w)).

### Comparative Example 16.

### Preparation of form A6, A7 and A8 of S1 (DMF-solvates)

**S1** (38 mg, Form A1) was slurried in 0.152 ml of DMF (Dimethylformamide) at 5 °C during 8 days. The sample was analyzed by XRPD showing that new forms A6, A7 and A8 occurred as the sample dried. TGA-data showed a weight loss between 40 - 180 °C of 16 % 8 (w/w) for form A6 and of 9 % (w/w) for form A7. Using NMR-analysis the material of form A7 was shown to contain 9.4 % (w/w) of DMF. These data indicate that form A6 is a mono-DMF solvate and form A7 is a hemi-DMF solvate.

### Example 17.

### Preparation of S1-2HCl form B1 from free base.

**S1** (3.00 g) was added to a round flask (100 ml) at room temperature (about 21°C). Ethanol (99.7%, 30 ml) was added. The substance was only partly dissolved. HCl (32%, 2.5 ml) corresponding to 3 equivalents was added to the slurry. The substance dissolved shortly but precipitated again as a gel. The gel was quickly transformed into a slurry of crystals. The slurry was left with stirring for 3 hours before it was filtered off. The filter cake was washed with 10 ml ethanol and dried under vacuum at 35 °C.. At total of 3.41 g of form I was obtained. The sample was analyzed by XRPD which showed that form B1 (as in Figure 9) had been obtained.

### Example 18.

### Preparation of S1-2HCl form B2 (dihydrate)

A solution of **S1-2HCl** (19.9mg, form B1) in water (1034 mg) was prepared. Then, **S1-2HCl** (23.9 mg, form B1) was added to 140 mg of the solution at 5°C. The resulting slurry was left stirred for 8 days before analysis. The sample was analyzed by XRPD which showed that form B2 (as in Figure 10) had been obtained. The slurry was filtered off and washed with a small amount of water. After drying in air the substance was reanalyzed with XRPD showing that still form B2 was present. TGA analysis showed a weight loss corresponding to 7.8 % (w/w). The substance was analyzed for HCl-content by ion-chromatography, which corresponded to a molar ratio of 1:1.8. (base:HCl). This indicates that a dihydrate- dihydrochloride salt had been obtained.

### Example 19.

### Purification by reformation and recrystallization of S1-2HCl from crude S1-2HCl.

Crude **S1-2HCl** (69.7g, 129.9 mmol), with a chromatographic purity of 79% at 210 nm, was suspended in water (300 ml) and 2-methyltetrahydrofuran (290 ml). The mixture was warmed to 30°C and the pH was adjusted to 12.5 using 45% sodium hydroxide. The resulting two phase system was filtered, the phases separated and the aqueous phase was discarded. The organic phase was concentrated under reduced pressure and the residue was dissolved in ethanol (580 ml). Hydrochloric acid 37% (14 ml) was added drop wise forming a stiff gel which was de-lumped with a spoon. Upon heating the mixture to 75°C a slurry was obtained and hydrochloric acid 37% (15 ml) was added drop wise. A clear solution was obtained followed by the precipitation of the **S1-2HCl.** The formed slurry was cooled to 25°C and stirred overnight. The solids was isolated by filtration, washed with ethanol (100 ml) and dried under reduced pressure at 40°C giving **S1-2HCl** (51.4g, 119.0 mmol) with a chromatographic purity of 98.6% at 210 nm.

### Items of the invention

1. A crystal form of (R)-(2-(4-chlorophenyl)quinolin-4-yl)((S)-piperidin-2-yl)methanol in the form of an anhydrate (Form A1), wherein said crystal form has a powder X-ray diffraction pattern, as obtained with CuKα1-radiation, with characteristic peaks at diffraction angles (°2theta) of 10.4 ± 0.2 and 19.1 ± 0.2.
2. The crystal form of item 1, wherein said crystal form has a powder X-ray diffraction pattern, as obtained with CuKα1-radiation, with characteristic peaks at diffraction angles (°2theta) of 10.4 ± 0.2, 19.1 ± 0.2, 12.7 ± 0.2, 14.7 ± 0.2, 21.0 ± 0.2 and 23.0 ± 0.2.
3. The crystal form of item 1 or 2, wherein said crystal form has a powder X-ray diffraction pattern essentially as set out in Figure 1.
4. A crystal form of (R)-(2-(4-chlorophenyl)quinolin-4-yl)((S)-piperidin-2-yl)methanol in the form of adihydrate (Form A2), wherein said crystal form has a powder X-ray diffraction pattern, as obtained with CuKα1-radiation, with characteristic peaks at diffraction angles (°2theta) of 5.4 ± 0.2, 10.8 ± 0.2 and 19.4 ±0.2.
5. The crystal form of item 4, wherein said crystal form has a powder X-ray diffraction pattern, as obtained with CuKα1-radiation, with characteristic peaks at diffraction angles (°2theta) of 5.4 ± 0.2, 10.8 ± 0.2, 19.4 ± 0.2, 14.4 ± 0.2, 21.6 ± 0.2 and 23.9 ± 0.2.
6. The crystal form of item 4 or 5, wherein said crystal form has a powder X-ray diffraction pattern essentially as set out in Figure 2.
7. A crystal form of (R)-(2-(4-chlorophenyl)quinolin-4-yl)((S)-piperidin-2-yl)methanol in the form of an monohydrate (Form A5), wherein said crystal form has a powder X-ray diffraction pattern, as obtained with CuKα1-radiation, with characteristic peaks at diffraction angles (°2theta) of 9.2 ± 0.2, 10.3 ± 0.2 and 19.1 ±0.2.
8. The crystal form of item 7, wherein said crystal form has a powder X-ray diffraction pattern, as obtained with CuKα1-radiation, with characteristic peaks at diffraction angles (°2theta) of 9.2 ± 0.2, 10.3 ± 0.2, 19.1 ± 0.2, 8.3 ± 0.2, 16.6 ± 0.2 and 16.8 ± 0.2.
9. The crystal form of item 7 or 8, wherein said crystal form has a powder X-ray diffraction pattern essentially as set out in Figure 5.
10. A crystal form of (R)-(2-(4-chlorophenyl)quinolin-4-yl)((S)-piperidin-2-yl)methanol in the form of an anhydrate (Form A10), wherein said crystal form has a powder X-ray diffraction pattern, as obtained with CuKα1-radiation, with characteristic peaks at diffraction angles (°2theta) of 10.4 ± 0.2 and 19.1 ±0.2.
11. The crystal form of item 7, wherein said crystal form has a powder X-ray diffraction pattern, as obtained with CuKα1-radiation, with characteristic peaks at diffraction angles (°2theta) of 10.4 ± 0.2, 19.1 ± 0.2, 10.1 ± 0.2, 14.7 ± 0.2, 19.7 ± 0.2, 21.0 ± 0.2.
12. The crystal form of item 10 or 11, wherein said crystal form has a powder X-ray diffraction pattern essentially as set out in Figure 8.
13. A crystal form form of (R)-(2-(4-chlorophenyl)quinolin-4-yl)((S)-piperidin-2-yl)methanol dihydrochloride salt (Form B1), wherein said crystal form has a powder X-ray diffraction pattern, as obtained with CuKα1-radiation, with characteristic peaks at diffraction angles (°2theta) of 5.9 ± 0.2, 10.9 ± 0.2 and 26.1 ± 0.2.
14. The crystal form of item 13, wherein said crystal form has a powder X-ray diffraction pattern, as obtained with CuKα1-radiation, with characteristic peaks at diffraction angles (°2theta) of 5.9 ± 0.2, 10.9 ± 0.2, 26.1 ± 0.2, 15.0 ± 0.2, 24.9 ± 0.2 and 25.1 ± 0.2.
15. The crystalline form of item 13 or 14, wherein said crystal form has a powder X-ray diffraction pattern essentially as set out in Figure 9.
16. A crystal form form of (R)-(2-(4-chlorophenyl)quinolin-4-yl)((S)-piperidin-2-yl)methanol dihydrochloride salt (Form B2), wherein said crystal form has a powder X-ray diffraction pattern, as obtained with CuKα1-radiation, with characteristic peaks at diffraction angles (°2theta) of 7.1 ± 0.2 and 14.2 ± 0.2.
17. The crystal form of item 16, wherein said crystal form has a powder X-ray diffraction pattern, as obtained with CuKα1-radiation, with characteristic peaks at diffraction angles (°2theta) of 7.1 ± 0.2, 14.2 ± 0.2, 10.6 ± 0.2, 11.9 ± 0.2 and 21.2 ± 0.2.
18. The crystalline form of item 16 or 17, wherein said crystal form has a powder X-ray diffraction pattern essentially as set out in Figure 10.
19. A pharmaceutical composition comprising a crystal form according to any one of items 1 to 18, together with pharmaceutically acceptable excipients.
20. A method of preparing (R)-(2-(4-chlorophenyl)quinolin-4-yl)((S)-piperidin-2-yl)methanol (**S1**) in crystal form A1, according to any one of items 1 to 3, said method comprising:
   - dissolving amorphous or crystalline **S1** in a substantially water free organic solvent, at a temperature between 25 °C - 150 °C;
   - performing crystallization by cooling to room temperature, evaporation or the addition of an anti-solvent, or a combination of these methods, until a slurry of crystals in a satureted solution has been obtained;
   - stirring said slurry to allow metastable solvates to be transformed to form A1; and
   - filtering and drying said A1 until the sample is dry.
21. A method of preparing (R)-(2-(4-chlorophenyl)quinolin-4-yl)((S)-piperidin-2-yl)methanol (**S1**) in crystal form A2, according to any one of items 4 to 6, said method comprising:
   - dissolving amorphous or crystalline **S1** in water or a mixture of water in an organic solvent, at a temperature below 50 °C;
   - if spontaneous crystallization does not occur, performing crystallization by evaporation or the addition of an anti-solvent, or a combination of these, until a slurry of crystals in a satureted solution has been obtained;
   - stirring said slurry to allow metastable solvates to be transformed to form A2; and
   - filtering and drying said A2 until the sample is dry.
22. A method of preparing (R)-(2-(4-chlorophenyl)quinolin-4-yl)((S)-piperidin-2-yl)methanol (**S1**) in crystal form A5, according to any one of items 7 to 9, said method comprising:
   - complete dissolution of amorphous or crystalline **S1** in chloroform at reflux;
   - the heating is turned off such that the solution is allowed to evaporate and cool down, yielding crystals of form A3;
   - filtering and drying said A3 until all chloroform is removed;
   - The dried material is then exposing said dried A3 to humid air, at room temperature until form A5 is formed.
23. A method of preparing (R)-(2-(4-chlorophenyl)quinolin-4-yl)((S)-piperidin-2-yl)methanol 2HCl (**S1-2HCl**) in crystal form B1, according to any one of items 13 to 15, said method comprising:
   - dissolving amorphous or crystalline **S1-2HCl** in a substantially water free organic solvent, at a temperature between 25 - 150 °C;
   - if spontaneous crystallization does not occur, performing crystallization by cooling to room temperature, evaporation or the addition of an anti-solvent, or a combination of these methods, until a slurry of crystals in a saturated solution has been obtained; and
   - filtering off and drying said B1 crystals, until the crystals are dry.
24. A method of preparing (R)-(2-(4-chlorophenyl)quinolin-4-yl)((S)-piperidin-2-yl)methanol 2HCl (**S1-2HCl**) in crystal form B2, according to any one of items 16 to 18, said method comprising:
   - dissolving amorphous or crystalline **S1-2HCl** in water or a mixture of water in an organic solvent, at a temperature below 50 °C;
   - if spontaneous crystallization does not occur, performing crystallization by evaporation or the addition of an anti-solvent, or a combination of these, until a slurry of crystals in a satureted solution is obtained; and
   - filtering off and drying said crystals slowly at a sufficiently low temperature such that structurally bound water molecules are not lost.
25. A method of treating cancer in a mammal, the method comprising administering to the mammal a therapeutically effective amount of a crystal form according to any one of items 1 to 18.
26. A crystal form according to any one of items 1 to 18, for use in the treatment of cancer.
27. Use of a crystal form according to any one of items 1 to 18, in the preparation of a medicament useful in the treatment of cancer.

## Claims

1. A crystal form of (R)-(2-(4-chlorophenyl)quinolin-4-yl)((S)-piperidin-2-yl)methanol in the form of an anhydrate (Form A1), wherein said crystal form has a powder X-ray diffraction pattern, as obtained with CuKα1-radiation, with characteristic peaks at diffraction angles (°2theta) of 10.4 ±0.2 and 19.1 ±0.2.

2. The crystal form of claim 1, wherein said crystal form has a powder X-ray diffraction pattern essentially as set out in Figure 1.

3. A crystal form of (R)-(2-(4-chlorophenyl)quinolin-4-yl)((S)-piperidin-2-yl)methanol in the form of adihydrate (Form A2), wherein said crystal form has a powder X-ray diffraction pattern, as obtained with CuKα1-radiation, with characteristic peaks at diffraction angles (°2theta) of 5.4 ± 0.2, 10.8 ± 0.2 and 19.4 ± 0.2.

4. The crystal form of claim 3, wherein said crystal form has a powder X-ray diffraction pattern essentially as set out in Figure 2.

5. A crystal form of (R)-(2-(4-chlorophenyl)quinolin-4-yl)((S)-piperidin-2-yl)methanol in the form of an monohydrate (Form A5), wherein said crystal form has a powder X-ray diffraction pattern, as obtained with CuKα1-radiation, with characteristic peaks at diffraction angles (°2theta) of 9.2 ± 0.2, 10.3 ± 0.2 and 19.1 ±0.2.

6. The crystal form of claim 5, wherein said crystal form has a powder X-ray diffraction pattern essentially as set out in Figure 5.

7. A crystal form of (R)-(2-(4-chlorophenyl)quinolin-4-yl)((S)-piperidin-2-yl)methanol in the form of an anhydrate (Form A10), wherein said crystal form has a powder X-ray diffraction pattern, as obtained with CuKα1-radiation, with characteristic peaks at diffraction angles (°2theta) of 10.4 ±0.2 and 19.1 ±0.2.

8. The crystal form of claim 7, wherein said crystal form has a powder X-ray diffraction pattern essentially as set out in Figure 8.

9. A crystal form form of (R)-(2-(4-chlorophenyl)quinolin-4-yl)((S)-piperidin-2-yl)methanol dihydrochloride salt (Form B1), wherein said crystal form has a powder X-ray diffraction pattern, as obtained with CuKα1-radiation, with characteristic peaks at diffraction angles (°2theta) of 5.9 ± 0.2, 10.9 ± 0.2 and 26.1 ± 0.2.

10. The crystal form of claim 9, wherein said crystalline form has a powder X-ray diffraction pattern essentially as set out in Figure 9.

11. A crystal form form of (R)-(2-(4-chlorophenyl)quinolin-4-yl)((S)-piperidin-2-yl)methanol dihydrochloride salt (Form B2), wherein said crystal form has a powder X-ray diffraction pattern, as obtained with CuKα1-radiation, with characteristic peaks at diffraction angles (°2theta) of 7.1 ± 0.2 and 14.2 ± 0.2.

12. The crystal form of claim 11, wherein said crystalline form has a powder X-ray diffraction pattern essentially as set out in Figure 10.

13. A pharmaceutical composition comprising a crystal form according to anyone of claims 1 to 12, together with pharmaceutically acceptable excipients.

14. A method of preparing (R)-(2-(4-chlorophenyl)quinolin-4-yl)((S)-piperidin-2-yl)methanol (**S1**) in crystal form A1, according to claim 1 or 2, said method comprising:
• dissolving amorphous or crystalline **S1** in a substantially water free organic solvent, at a temperature between 25 °C - 150 °C;
• performing crystallization by cooling to room temperature, evaporation or the addition of an anti-solvent, or a combination of these methods, until a slurry of crystals in a satureted solution has been obtained;
• stirring said slurry to allow metastable solvates to be transformed to form A1; and
• filtering and drying said A1 until the sample is dry.

15. A method of preparing (R)-(2-(4-chlorophenyl)quinolin-4-yl)((S)-piperidin-2-yl)methanol (**S1**) in crystal form A2, according to claim 3 or 4, said method comprising:
• dissolving amorphous or crystalline **S1** in water or a mixture of water in an organic solvent, at a temperature below 50 °C;
• if spontaneous crystallization does not occur, performing crystallization by evaporation or the addition of an anti-solvent, or a combination of these, until a slurry of crystals in a satureted solution has been obtained;
• stirring said slurry to allow metastable solvates to be transformed to form A2; and
• filtering and drying said A2 until the sample is dry.

16. A method of preparing (R)-(2-(4-chlorophenyl)quinolin-4-yl)((S)-piperidin-2-yl)methanol (**S1**) in crystal form A5, according to claim 5 or 6, said method comprising:
• complete dissolution of amorphous or crystalline **S1** in chloroform at reflux;
• the heating is turned off such that the solution is allowed to evaporate and cool down, yielding crystals of form A3;
• filtering and drying said A3 until all chloroform is removed;
• The dried material is then exposing said dried A3 to humid air, at room temperature until form A5 is formed.

17. A method of preparing (R)-(2-(4-chlorophenyl)quinolin-4-yl)((S)-piperidin-2-yl)methanol 2HCl (**S1-2HCl**) in crystal form B1, according to claim 9 or 10, said method comprising:
• dissolving amorphous or crystalline **S1-2HCl** in a substantially water free organic solvent, at a temperature between 25 - 150 °C;
• if spontaneous crystallization does not occur, performing crystallization by cooling to room temperature, evaporation or the addition of an anti-solvent, or a combination of these methods, until a slurry of crystals in a saturated solution has been obtained; and
• filtering off and drying said B1 crystals, until the crystals are dry.

18. A method of preparing (R)-(2-(4-chlorophenyl)quinolin-4-yl)((S)-piperidin-2-yl)methanol 2HCl (**S1-2HCl**) in crystal form B2, according to claim 11 or 12, said method comprising:
• dissolving amorphous or crystalline **S1-2HCl** in water or a mixture of water in an organic solvent, at a temperature below 50 °C;
• if spontaneous crystallization does not occur, performing crystallization by evaporation or the addition of an anti-solvent, or a combination of these, until a slurry of crystals in a satureted solution is obtained; and
• filtering off and drying said crystals slowly at a sufficiently low temperature such that structurally bound water molecules are not lost.

19. A crystal form according to any one of claims 1 to 12, for use in the treatment of cancer.
